# EUROPÄISCHE PATENTSCHRIFT

(11) **EP 0 510 444 B1**
(45) Veröffentlichungstag und Bekanntmachung des Hinweises auf die Patenterteilung: **01.02.1995**
(21) Anmeldenummer: 92106140.4
(22) Anmeldetag: 09.04.1992
(51) Int. Cl.: C07C 309/73, G03F 7/039

(54) **Säurespaltbare, strahlungsempfindliche Verbindungen, diese enthaltendes strahlungsempfindliches Gemisch und daraus hergestelltes strahlungsempfindliches Aufzeichnungsmaterial**
Acid-cleavable radiation-sensitive compounds, radiation-sensitive composition containing the same and radiation-sensitive recording material prepared therefrom
Composés radiosensibles clivables par l'acide, composition radiosensible les contenant et matériau d'enregistrement radiosensible ainsi obtenu

(30) Priorität: 20.04.1991 DE 4112969
(43) Veröffentlichungstag der Anmeldung: 28.10.1992
(73) Patentinhaber: HOECHST AKTIENGESELLSCHAFT, 65926 Frankfurt am Main (DE)
(72) Erfinder: Pawlowski, Georg, Dr., W-6200 Wiesbaden (DE); Dammel, Ralph, Dr., Coventry, Rhode Island 02816 (US); Röschert, Horst, Dr., W-6531 Ober Hilbersheim (DE); Spiess, Walter, Dr., W-6110 Dieburg (DE); Eckes, DI Charlotte, W-6200 Wiesbaden-Dotzheim (DE)

(56) Entgegenhaltungen:
- EP-A- 0 297 443
- DE-A- 2 718 254
- MICROELECTRONIC ENINEERING, Band 13, Nr. 1/4, März 1991, Seiten 33-36, Amsterdam, NL; L. SCHLEGEL et al.: "Highly sensitive positive deep UV resist utilizing a sulfonate acid generator and a tetrahydropyranyl inhibitor"
- MICROELECTRONIC ENINEERING, Band 13, Nr. 1/4, März 1991, Seiten 3-10, Amsterdam, NL; E. REICHMANIS et al.: "Chemistry and processes for deep-UV resists"

## Beschreibung

Die Erfindung betrifft strahlungsempfindliche und säurespaltbare Verbindungen sowie ein positiv arbeitendes, d.h. durch Bestrahlung löslich werdendes strahlungsempfindliches Gemisch, das diese Verbindungen enthält. Das Gemisch enthält
(a) ein in Wasser unlösliches, in wäßrig-alkalischen Lösungen lösliches oder zumindest quellbares Bindemittel und
(b) eine Verbindung, die unter Einwirkung von aktinischer Strahlung eine starke Säure bildet und die mindestens eine durch Säure spaltbare C-O-C-Bindung aufweist.

Die Erfindung betrifft weiterhin ein daraus hergestelltes strahlungsempfindliches Aufzeichnungsmaterial, das zur Herstellung von Photoresists, elektronischen Bauteilen, Druckplatten oder zum Formteilätzen geeignet ist.

Die stetige Verkleinerung der Strukturen, beispielsweise in der Chip-Herstellung bis in den Bereich von weniger als 1 »m, erfordert veränderte lithographische Techniken. Um solche feinen Strukturen abzubilden, verwendet man Strahlung mit einer kurzen Wellenlänge, wie energiereiches UV-Licht, Elektronen- und Röntgenstrahlen. Das strahlungsempfindliche Gemisch muß an die kurzwellige Strahlung angepaßt sein. Eine Zusammenstellung der an das strahlungsempfindliche Gemisch gestellten Anforderungen ist in dem Aufsatz von C.G. Willson "Organic Resist Materials - Theory and Chemistry" [Introduction to Microlithography, Theory, Materials, and Processing, Herausgeber L.F. Thompson, C.G. Willson, M.J. Bowden, ACS Symp. Ser., 219, 87 (1983), American Chemical Society, Washington] angegeben. Es bestand daher ein verstärkter Bedarf an strahlungsempfindlichen Gemischen, die in den neueren Technologien, wie der Mid- oder Deep-UV-Lithographie, [Belichtung z. B. mit Excimer-Lasern bei Wellenlängen von 305 nm (XeF), 248 nm (KrF), 193 nm (ArF)], der Elektronen- oder der Röntgenstrahl-Lithographie, einsetzbar sind, vorzugsweise darüber hinaus in einem weiten Spektralbereich empfindlich sind und dementsprechend auch in der konventionellen UV-Lithographie verwendet werden können.

Positiv arbeitende strahlungsempfindliche Gemische zur Herstellung strahlungsempfindlicher Aufzeichnungsmaterialien sind bekannt. Handelsüblich sind Gemische, die o-Chinon-diazid-Derivate in wäßrig-alkalisch löslichen Bindemitteln, beispielsweise Novolaken oder Polyhydroxystyrolen, enthalten. Die Empfindlichkeit dieser Materialien gegenüber aktinischer, insbesondere aber hochenergetischer, kurzwelliger Strahlung, wie Licht eines KrF-Excimer-Lasers mit einer Wellenlänge von 248 nm oder Elektronenstrahlung, ist aber unzureichend.

Weiterhin sind positiv arbeitende strahlungsempfindliche Gemische bekannt, bei denen durch Einwirkung von aktinischer Strahlung auf einen in dieser Mischung enthaltenen Photoinitiator eine Säure gebildet wird und diese dann in einer Folgereaktion eine ebenfalls in der Mischung enthaltene säurespaltbare Verbindung in den bestrahlten Bereichen gegenüber einem entsprechenden, bevorzugt wäßrig-alkalischen Entwickler löslich macht. Derartige Materialien zeichnen sich im allgemeinen durch eine verbesserte Empfindlichkeit gegenüber aktinischer Strahlung aus.

Es sind zahlreiche Gemische bekannt, die als wesentliche Komponenten ein polymeres, in wäßrig-alkalischen Lösungen lösliches Bindemittel, eine löslichkeitsinhibierende Verbindung und eine Verbindung, die bei Bestrahlung die zur Spaltung erforderliche Säure liefert, enthalten. Das Bindemittel ist zumeist ein Novolak-Harz. Viele dieser Gemische besitzen eine hohe Empfindlichkeit gegenüber aktinischer Strahlung. Sie werden als chemisch verstärkte, photokatalytische 3-Komponentensysteme bezeichnet.

Von diesen Gemischen haben insbesondere solche eine gewisse kommerzielle Bedeutung erlangt, deren säurespaltbare Komponente eine oder mehrere Acetaleinheiten aufweist. Diese Gemische haben jedoch gewisse Nachteile. So besitzen sie auf den Substratmaterialien, auf die sie aufgebracht werden müssen, eine nur begrenzte Stabilität, was zu einer unzureichenden, nicht reproduzierbaren Wiedergage der Bildvorlage führt. Dies kann nur durch Einführung zusätzlicher Schutzschichten, z. B. gemäß DE-A 36 21 376 (= US-A 4 840 867), verbessert werden. Die Ursachen der Verschlechterung der Bildwiedergabe sind nicht im einzelnen bekannt und nur unzureichend untersucht. Beispielsweise ist das Prozeßfenster, d. h. der Verarbeitungsspielraum, für die Belichtung dieser Gemische sehr schmal und häufig nicht eindeutig reproduzierbar. Insbesondere hängt die Qualität der Bildwiedergabe stark von der Zeitdifferenz zwischen Belichtung und Entwicklung, der sogenannten "delay time" ab. Grundsätzlich ist davon auszugehen, daß Diffusionsvorgänge, die zu diesem Verhalten führen, nicht einfach steuerbar sind. Es läßt sich jedoch zusätzlich vermuten, daß es während der Trocknung des Gemischs auf einem Substratmaterial zu einer partiellen Verdampfung des Photoinitiators oder der säurelabilen Verbindung oder zu einer Entmischung der einzelnen Gemischbestandteile kommt, was besonders häufig bei säurelabilen Verbindungen mit geringer Löslichkeit in den üblichen Beschichtungslösemitteln beobachtet wird.

Ferner ist durch die Arbeiten von C.G. Petropoulos [J. Polym. Sci., Polym. Chem. Ed., 15, 1637 (1977)] bekannt, daß aromatische Acetale, die in Nachbarstellung eine Nitrogruppe tragen, ohne Säurekatalyse durch energiereiche UV-Strahlung photozersetzbar sind, und in positiv arbeitenden strahlungsempfindlichen Aufzeichnungsmaterialien Verwendung finden können. Die Photoempfindlichkeit dieser Verbindungen ist ebenfalls für praktische Anwendungen unzureichend, da ihre Lichtreaktion nicht chemisch verstärkt werden kann.

In der DE-A 37 21 741 (= US-A 4 883 740) werden strahlungsempfindliche Gemische beschrieben, die ein wasserunlösliches, in wäßrig-alkalischen Lösungen lösliches polymeres Bindemittel und eine organische Verbindung enthalten, die mindestens eine durch Säure spaltbare Gruppierung sowie eine Gruppierung enthält, die unter Einwirkung von Strahlung eine starke Säure bildet. Als strahlungsempfindliche Gruppen werden ausschließlich Oniumsalzgruppen, insbesondere Sulfoniumsalzgruppen, beschrieben.

Die Verwendung von Onium-Salzen, wie Diazonium-, Phosphonium-, Sulfonium- und Jodonium-Salze von nicht nucleophilen Säuren, wie HSbF₆, HAsF₆ oder HPF₆ als photolytische Säurebildner, bringt Nachteile mit sich, die ihre Einsatzmöglichkeiten in verschiedenen Anwendungsbereichen drastisch einschränken. So sind z.B. viele der Oniumsalze toxisch. Ihre Löslichkeit ist in vielen Lösemitteln unzureichend, weshalb nur wenige Lösemittel zur Herstellung einer Beschichtungslösung geeignet sind. Darüber hinaus werden bei Verwendung der Oniumsalze z.T. unerwünschte Fremdatome eingeführt, die insbesondere in der Mikrolithographie zu Prozeßstörungen führen können. Ferner bilden die Oniumsalze bei der Photolyse sehr stark korrodierend wirkende Brönstedt-Säuren. Diese Säuren greifen empfindliche Substrate an, so daß der Einsatz solcher Gemische zu unbefriedigenden Ergebnissen führt. Auch Halogenverbindungen, wie Trichlormethyltriazin-Derivate oder Trichlormethyloxadiazol-Derivate, bilden stark korrosiv wirkende Halogenwasserstoffsäuren.

In neueren Arbeiten von F.M. Houlihan et al., SPIE 920, 67 (1988) wurden anhand positiv arbeitender Systeme gezeigt, daß neben den oben genannten Säurebildnern auch Nitrobenzyltosylate, die bei Belichtung Sulfonsäuren mit geringer Wanderungstendenz bilden, in bestimmten säurelabilen Resistformulierungen verwendbar sind. Es kann aus diesen Ergebnissen abgeleitet werden, daß solche Verbindungen auch für photohärtbare Systeme verwendet werden können. Die dabei erzielten Empfindlichkeiten, insbesondere gegenüber UV-Strahlung von 350 bis 450 nm und die thermische Stabilität der Photoresists erwiesen sich jedoch als unzureichend.

Wegen der aufgeführten Unzulänglichkeiten und Nachteile besteht daher ein Bedarf an weiteren strahlungsempfindlichen Gemischen, die die oben beschriebenen Nachteile nicht aufweisen und eine praxisgerechte Reaktivität besitzen.

Aufgabe der Erfindung war es daher, photolytisch säurebildende und säurespaltbare Verbindungen sowie ein strahlungsempfindliches Gemisch auf deren Basis vorzuschlagen, wobei die photolytisch eine Säure bildende Verbindung möglichst stabil auf allen bekannten Substraten sein sollte und als Photolyseprodukt eine nicht korrosiv wirkende Säure liefert. Darüber hinaus soll mit der Erfindung ein strahlungsempfindliches Gemisch bereitgestellt werden, das insbesondere eine Entmischung der photoaktiven Verbindung und der löslichkeitsdifferenzierenden Verbindung vermeidet.

Erfindungsgemäß werden Verbindungen der allgemeinen Formel I 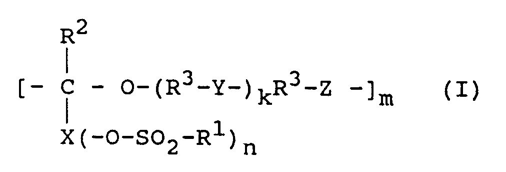
vorgeschlagen, worin
- R¹: einen Alkyl-, Halogenalkyl- oder Arylrest,
- R²: ein Wasserstoffatom, einen Alkyl-, Alkenyl- oder Arylrest oder die Gruppe (R¹-SO₂-O-)ₙX-,
- R³: eine Cycloalkylendialkyl-, Cycloalkenylendialkyl-, Arylendialkyl- oder Heteroarylendialkylgruppe, eine Alkylen-, Alkenylen-, Alkinylen-, Cycloalkylen- oder Arylengruppe bedeutet,
- X: eine mit 1 bis 3 Sulfonyloxygruppen R¹-SO₂-O substituierte Alkyl-, Cycloalkyl- oder Arylgruppe,
- Y: O, S, CO, CO-O, SO₂, SO₂-O, NR⁴, CO-NH, O-CO-NR⁵, NH-CO-NR⁵ oder NR⁵-CO-O,
- Z: O, CO-NR⁶, O-CO-NR⁶ oder NH-CO-NR⁶,
- R⁴: einen Acylrest,
- R⁵: ein Wasserstoffatom, einen Alkyl-, Cycloalkyl, Alkenyl-, Alkinyl- oder Arylrest,
- R⁶: einen Alkyl-, Cycloalkyl, Alkenyl-, Alkinyl- oder Arylrest,
- k: eine ganze Zahl von 0 bis 4,
- m: eine ganze Zahl größer als 1 und
- n: eine ganze Zahl von 1 bis 3
bedeuten, wobei R³ und Y in sich wiederholenden Gruppierungen (R³-Y-) gleiche oder unterschiedliche Bedeutungen haben können.
Bevorzugt sind solche Verbindungen der allgemeinen Formel I, in denen R¹ einen gegebenenfalls halogenierten, insbesondere fluorierten Alkylrest mit 1 bis 6 Kohlenstoffatomen oder einen Arylrest mit 6 bis 12 Kohlenstoffatomen, insbesondere einen einkernigen Arylrest bedeutet. Der Arylrest kann 1 bis 3 Substituenten tragen, insbesondere Halogenatome, Nitrogruppen, Cyanogruppen, Alkyl- oder Alkoxygruppen mit 1 bis 3 Kohlenstoffatomen.

R² ist bevorzugt ein Wasserstoffatom, ein Alkylrest mit 1 bis 6, insbesondere 1 bis 3, Kohlenstoffatomen, ein Alkenylrest mit 2 bis 6, insbesondere 2 bis 4, Kohlenstoffatomen oder ein Arylrest mit 6 bis 12 Kohlenstoffatomen, der gegebenenfalls durch Halogenatome, Alkylreste mit 1 bis 3 Kohlenstoffatomen, Alkansulfonyloxyreste mit 1 bis 5 Kohlenstoffatomen oder Alkoxyreste mit 1 bis 3 Kohlenstoffatomen substituiert ist, oder ein Rest der Formel (R¹-SO₂O)ₙX, besonders bevorzugt ein Wasserstoffatom.

R³ ist bevorzugt eine Cycloalkenylendialkyl-, Cycloalkylendialkyl-, Arylendialkyl- oder Heteroylendialkylgruppe mit 6 bis 20, insbesondere 6 bis 15, Kohlenstoffatomen, eine Alkylengruppe mit 1 bis 8, eine Alkenylen- oder Alkinylengruppe mit 2 bis 10, insbesondere 2 bis 6 Kohlenstoffatomen, eine Cycloalkylengruppe mit 4 bis 12 oder eine Arylengruppe mit 6 bis 10, insbesondere eine einkernige Arylengruppe mit 6 bis 8 Kohlenstoffatomen.

X ist bevorzugt eine Alkylgruppe mit 2 bis 8 Kohlenstoffatomen, eine Cycloalkylgruppe mit 5 bis 8 Kohlenstoffatomen oder eine Arylgruppe mit 6 bis 12, insbesondere eine einkernige Arylgruppe mit 6 bis 10 Kohlenstoffatomen, wobei Arylgruppen besonders bevorzugt werden. Diese Gruppen sind jeweils mit 1 bis 3 Sulfonyloxygruppen R¹-SO₂-O- substituiert.

Y ist bevorzugt O, S, NR⁴, SO₂, O-CO-NR⁵, CO-O, NH-CO-NR⁵ oder NR⁵-CO-O.

Die Gruppen R³ und Y können in den Fällen, in denen k größer als 1 ist, in jeder Wiederholung die gleiche oder eine unterschiedliche Bedeutung haben.

R⁴ ist bevorzugt ein Alkanoylrest mit 2 bis 6 oder ein Aroylrest mit 7 bis 10 Kohlenstoffatomen. Es kann auch der Rest einer anderen Säure, z. B. ein Sulfonyl- oder Phosphonylrest sein. Aroylreste sind besonders bevorzugt.
R⁵ ist bevorzugt ein Wasserstoffatom, ein Alkylrest mit 1 bis 8, insbesondere 1 bis 5, ein Alkenyl- oder Alkinylrest mit 2 bis 6 oder ein Arylrest mit 6 bis 10, insbesondere 6 bis 8 Kohlenstoffatomen.

R⁶ ist bevorzugt ein Alkylrest mit 1 bis 8, insbesondere 1 bis 5, ein Alkenyl- oder Alkinylrest mit 2 bis 6 oder ein Arylrest mit 6 bis 10, insbesondere 6 bis 8 Kohlenstoffatomen.

m ist bevorzugt eine ganze Zahl größer als 3.

Besonders bevorzugt sind solche Verbindungen der allgemeinen Formel I, in denen R¹ einen Methyl-, Ethyl-, Trifluormethyl- oder 1,1,2,3,3,3-Hexafluorpropylrest oder einen gegebenenfalls durch 1 bis 3 Alkyl- oder Alkoxygruppen mit 1 bis 3 Kohlenstoffatomen, 1 bis 3 Halogenatome, 1 oder 2 Nitro-, Cyano- oder Trifluormethylgruppen oder entsprechende Kombinationen substituierten Phenylrest darstellt.

Die Herstellungsweise solcher Verbindungen ist an sich bekannt. Sie kann beispielsweise analog zu den in der DE-A 26 10 842 angegebenen Synthesevorschriften erfolgen, wobei in einer Vorstufe das entsprechende Sulfonsäureesterderivat hergestellt werden muß. Im Folgenden wird die Synthese typischer und bevorzugter Vertreter dieser Verbindungsklassen an einzelnen Beispielen beschrieben.

### Herstellungsbeispiel 1

1. Stufe: 80,6 g (0,66 mol) 4-Hydroxybenzaldehyd wurden in 200 ml Tetrahydrofuran gelöst, mit 138 ml Triethylamin versetzt und auf -5 °C abgekühlt. Dazu wurden 125,8 g (0,66 mol) p-Toluolsulfonsäurechlorid, gelöst in 330 ml Tetrahydrofuran und auf -5 °C vorgekühlt, bei -8 bis -5 °C zugetropft. Es wurde 1 Stunde bei Raumtemperatur nachgerührt. Die Lösung wurde in 1000 ml destilliertes Wasser eingegossen und die Mischung mit konzentrierter Salzsäure auf pH 2 gebracht. Das ausgefallene Öl wurde in Ether aufgenommen und die wäßrige Phase mit Ether extrahiert. Die vereinigten organischen Phasen wurden mit Wasser gewaschen und getrocknet. Nach dem Einengen verblieb ein Rückstand, der aus Cyclohexan/Methylenchlorid umkristallisiert wurde. Es wurden 120 g (66,1%) 4-(Toluol-4-sulfonyloxy)-benzaldehyd (weißer Feststoff mit einem Schmelzpunkt von 70 bis 72 °C) erhalten.
2. Stufe: 7,2 g (0,026 mol) 4-(Toluol-4-sulfonyloxy)-benzaldehyd wurden zusammen mit 2,4 g (0,026 mol) Butan-1,4diol, 4,25 g (0,0286 mol) Orthoameisensäuretriethylester und 100 mg p-Toluolsulfonsäure in 150 ml Toluol zum Rückfluß erwärmt. Nach etwa 2 Stunden wurde ein Teil des Destillats abgenommen, bis die Kopftemperatur der Siedetemperatur des reinen Toluols entsprach. Dieser Vorgang wurde so lange wiederholt, bis die Kopftemperatur nicht mehr unter den Siedepunkt von reinem Toluol sank. Nach weiteren 2 Stunden wurde die Destillation für etwa 45 Minuten durch Anlegen eines Vakuums von 4 Torr unterstützt und alle flüchtigen Bestandteile abdestilliert. Der erkaltete Rückstand wurde in Methylenchlorid aufgenommen, je zweimal mit 2%iger Natronlauge und vollentsalztem Wasser gewaschen und die organische Phase getrocknet. Nach Entfernung des Lösemittels verblieben 9,1 g eines gelben Öls, das sich aufgrund seines NMR-Spektrums als die gewünschte Verbindung 1 erwies.

### Herstellungsbeispiel 2

1. Stufe: 80,6 g (0,66 mol) 3-Hydroxy-benzaldehyd wurden in 200 ml Tetrahydrofuran gelöst, mit 138 ml Triethylamin versetzt und auf -5 °C gekühlt. Dazu wurden 125,8 g (0,66 mol) p-Toluolsulfonsäurechlorid, gelöst in 330 ml Tetrahydrofuran und auf -5 °C vorgekühlt, bei -8 bis - 5 °C zugetropft. Es wurde 1 Stunde bei Raumtemperatur nachgerührt. Die Lösung wurde in 1000 ml destilliertes Wasser eingegossen und die Mischung mit konzentrierter Salzsäure auf pH 2 gebracht. Das ausgefallene Öl wurde in Ether aufgenommen und die wäßrige Phase mit Ether extrahiert. Die vereinigten organischen Phasen wurden mit Wasser gewaschen und getrocknet. Nach dem Einengen verblieb ein Öl, das aus Cyclohexan umkristallisiert wurde. Es wurden 115 g (63,7%) 3-(Toluol-4-sulfonyloxy)-benzaldehyd (weißer Feststoff mit einem Schmelzpunkt von 65 bis 67 °C) erhalten.
2. Stufe: 7,2 g (0,026 mol) 3-(Toluol-4-sulfonyloxy)-benzaldehyd wurden zusammen mit 4,15 g (0,026 mol) Butin-1,4-diol, 4,25 g (0,0286 mol) Orthoameisensäuretriethylester und 100 mg p-Toluolsulfonsäure in 150 ml Toluol im Ölbad auf 130 °C erwärmt. Nach etwa 2 Stunden wurde ein Teil des Destillats abgenommen, bis die Kopftemperatur der Siedetemperatur des reinen Toluols entsprach. Dieser Vorgang wurde so lange wiederholt, bis die Kopftemperatur nicht mehr unter den Siedepunkt von reinem Toluol sank. Nach weiteren 2 Stunden wurde die Destillation etwa 45 Minuten durch Anlegen eines Vakuums von 4 Torr unterstützt, und alle flüchtigen Bestandteile wurden abdestilliert. Der erkaltete Rückstand wurde in Methylenchlorid aufgenommen, je zweimal mit 2%iger Natronlauge und vollentsalztem Wasser gewaschen und die organische Phase getrocknet. Nach Entfernung des Lösemittels verblieben 9,8 g einer dunklen, bernsteinfarbenen Schmelze, die sich als die Verbindung 2 erwies.

### Herstellungsbeispiel 3

1. Stufe: 8,29 g (0,06 mol) 3,4-Dihydroxybenzaldehyd wurden in 40 ml Tetrahydrofuran gelöst, mit 18,5 ml Triethylamin versetzt und auf -5 °C gekühlt. Dazu wurden 22,9 g (0,12 mol) p-Toluolsulfonsäurechlorid, gelöst in 80 ml Tetrahydrofuran und auf -5 °C vorgekühlt, bei -8 bis -5 °C zugetropft. Es wurde 2 Stunden bei Raumtemperatur nachgerührt. Die Lösung wurde in 1000 ml destilliertes Wasser eingegossen und die Mischung mit konzentrierter Salzsäure auf pH 2 gebracht. Das ausgefallene Öl erstarrte nach längerem Rühren zu einem braunen Kristallbrei. Das Rohprodukt wurde abgenutscht und getrocknet. Es wurde aus Cyclohexan/Methylenchlorid unter Zusatz von Aktivkohle umkristallisiert. Es wurden 15,9 g (59,4%) 3,4-Bis-(toluol-4-sulfonyloxy)-benzaldehyd (weißer Feststoff mit einem Schmelzpunkt von 95 bis 97 °C) erhalten.
2. Stufe: 11,6 g (0,026 mol) 3,4-Bis-(toluol-4-sulfonyloxy)-benzaldehydder wurden zusammen mit 3,75 g (0,026 mol) Triethylenglykol, 4,25 g (0,0286 mol) Orthoameisensäuretriethylester und 100 mg p-Toluolsulfonsäure in 150 ml Toluol im Ölbad auf 130 °C erwärmt. Nach etwa 2 Stunden wurde ein Teil des Destillats abgenommen, bis die Kopftemperatur der Siedetemperatur des reinen Toluols entsprach. Dieser Vorgang wurde so lange wiederholt, bis die Kopftemperatur nicht mehr unter den Siedepunkt von reinem Toluol sank. Nach weiteren 2 Stunden wurde die Destillation für etwa 45 Minuten durch Anlegen eines Vakuums von 4 Torr unterstützt, und alle flüchtigen Bestandteile wurden abdestilliert. Der erkaltete Rückstand wurde in Methylenchlorid aufgenommen, je zweimal mit 2%iger Natronlauge und vollentsalztem Wasser gewaschen und die organische Phase getrocknet. Nach Entfernung des Lösemittels verblieben 13,8 g einer dunklen, honigartigen Flüssigkeit, die sich als die Verbindung 3 erwies.

### Herstellungsbeispiel 4

7,2 g (0,026 mol) 4-(Toluol-4-sulfonyloxy)-benzaldehyd (s. Herstellungsbeispiel 1, 1. Stufe) wurden zusammen mit 5,2 g (0,035 mol) Orthoameisensäuretriethylester, und 8,82 g (0,06 mol) eines Urethanalkohols, hergestellt durch Umsetzung von 1 mol Ethylencarbonat mit 1 mol n-Propylamin, und 100 mg p-Toluolsulfonsäure in 150 ml Toluol unter Rückfluß erhitzt. Nach etwa 2 Stunden wurde ein Teil des Destillats abgenommen, bis die Kopftemperatur der Siedetemperatur des reinen Toluols entsprach. Dieser Vorgang wurde so lange wiederholt, bis die Kopftemperatur nicht mehr unter den Siedepunkt von reinem Toluol sank. Nach weiteren 2 Stunden wurde die Destillation für etwa 45 Minuten durch Anlegen eines Vakuums von 4 Torr unterstützt, und alle flüchtigen Bestandteile wurden abdestilliert. Der erkaltete Rückstand wurde in Methylenchlorid aufgenommen, je zweimal mit 2%iger Natronlauge und vollentsalztem Wasser gewaschen und die organische Phase getrocknet. Nach Entfernung des Lösemittels verblieben 10,8 g einer hellen, viskosen Flüssigkeit, die einer Vakuumbehandlung bei 180 °C/0,01 Torr unterzogen wurde. Dabei wurde eine farblose Flüssigkeit abgespalten. Der verbliebene hochviskose Rückstand erwies sich als die Verbindung 4.

Weitere Beispiele von erfindungsgemäßen Verbindungen sind in den beiliegenden Formeln 5 bis 49 aufgeführt. 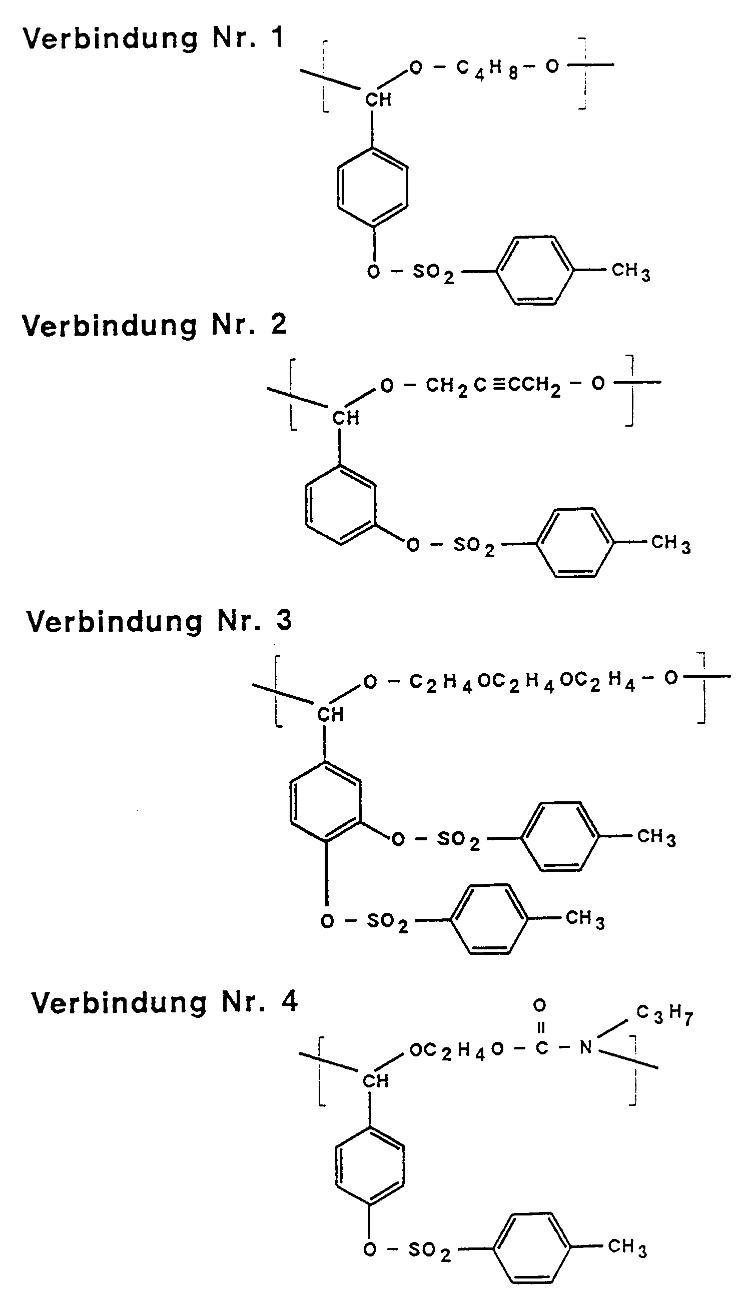 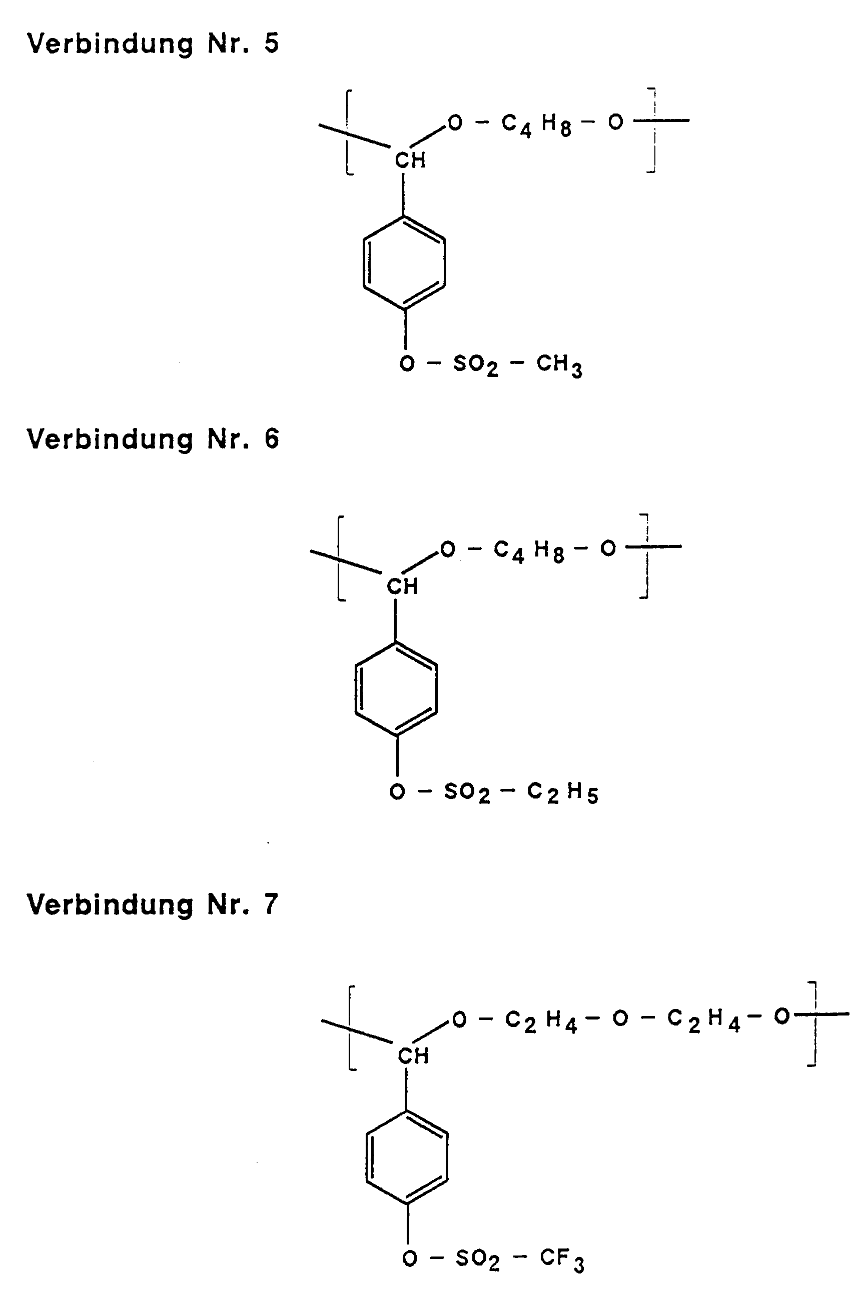 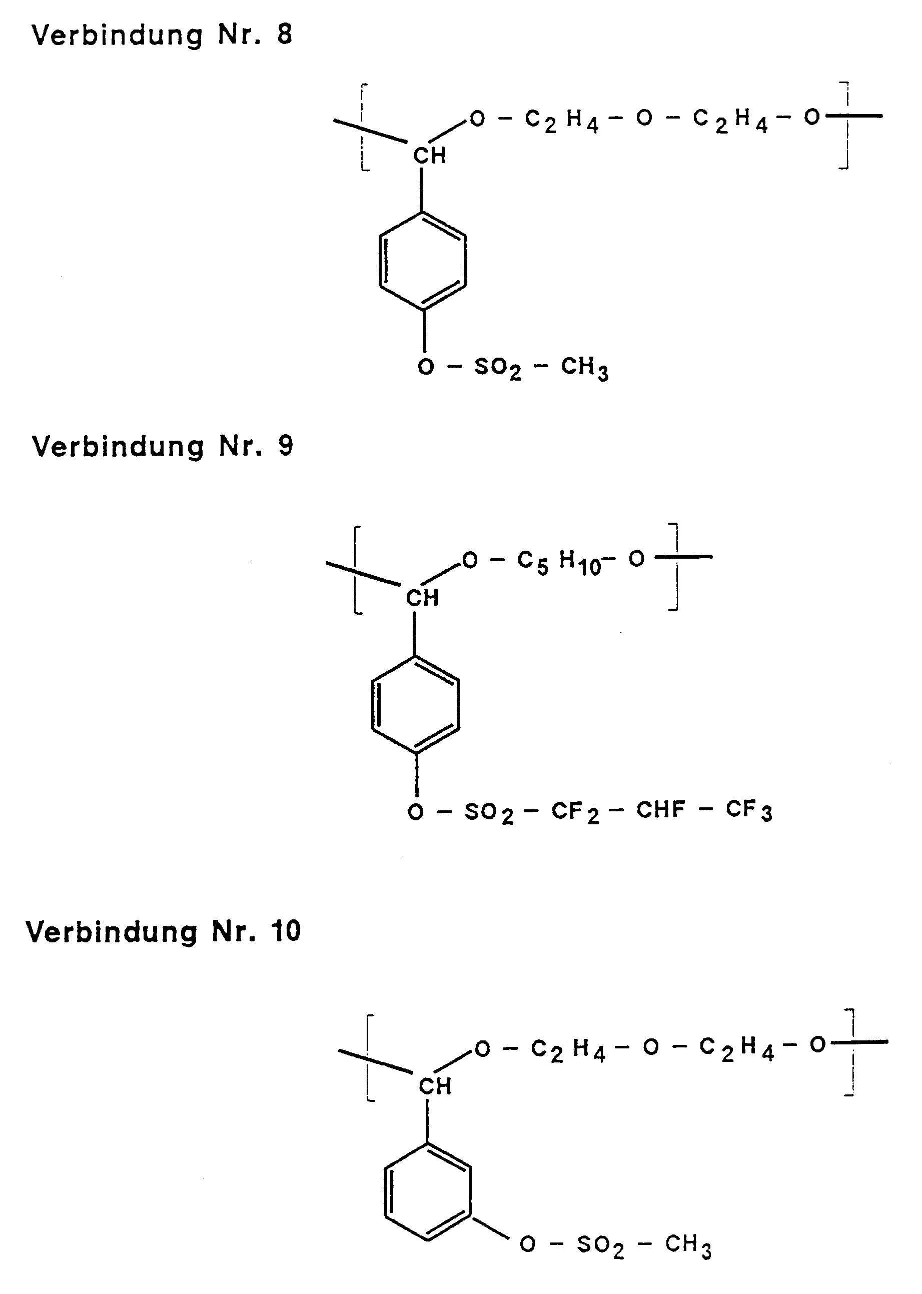 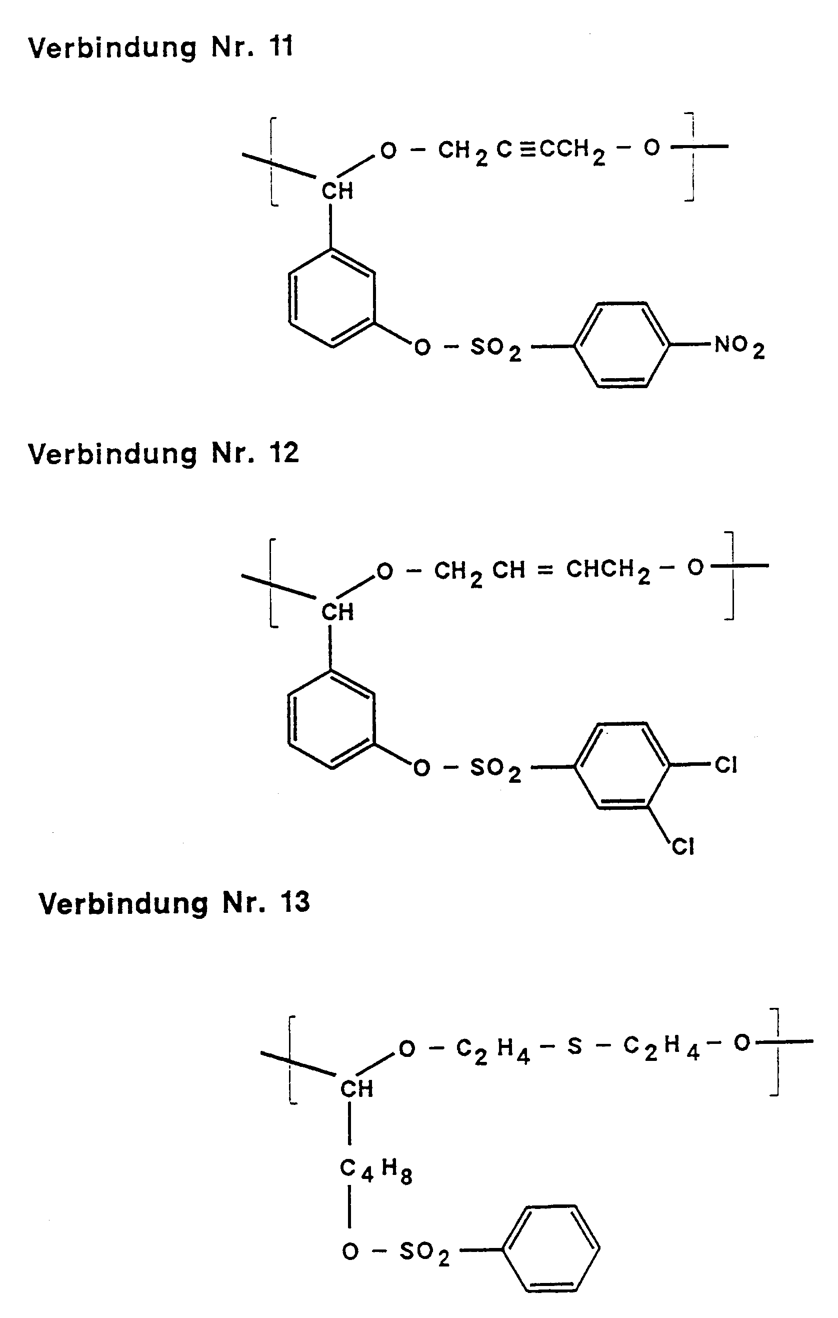 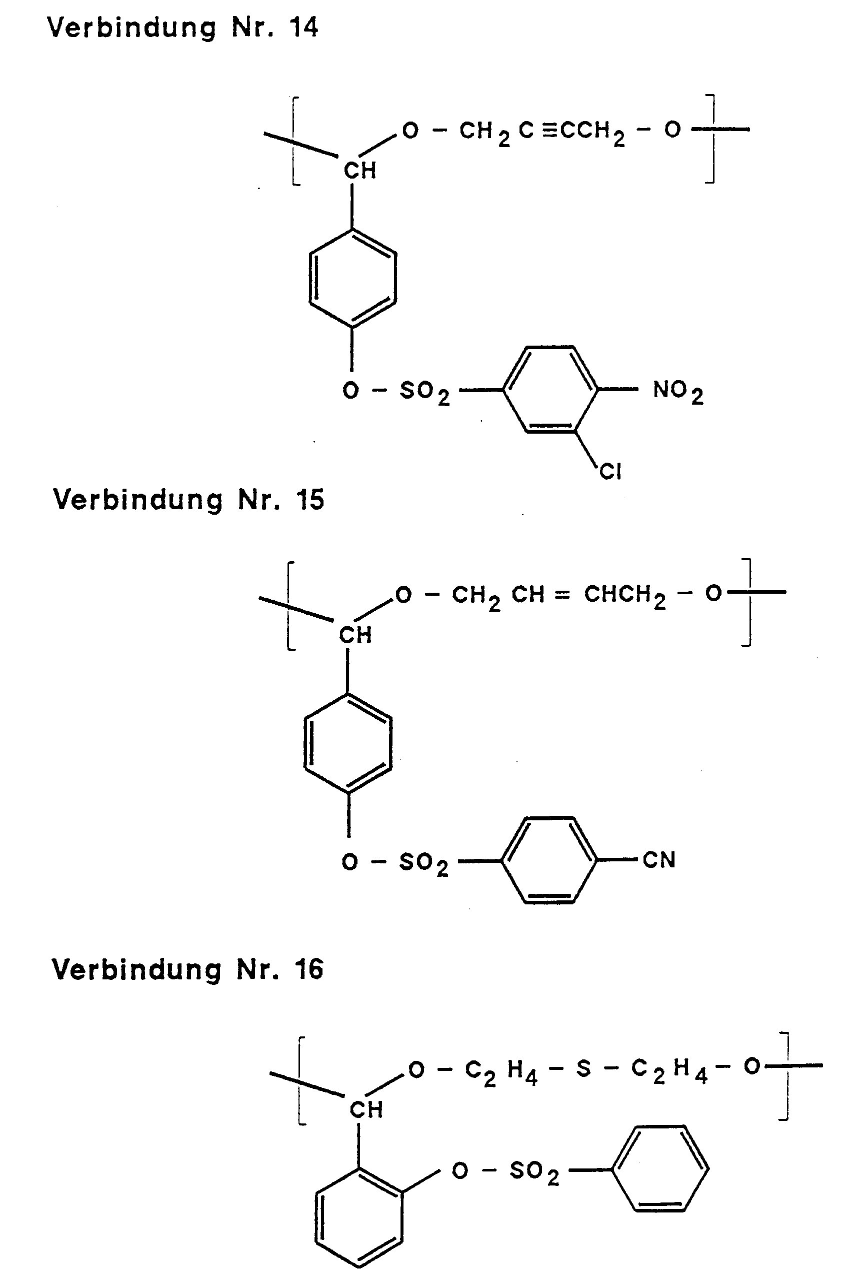 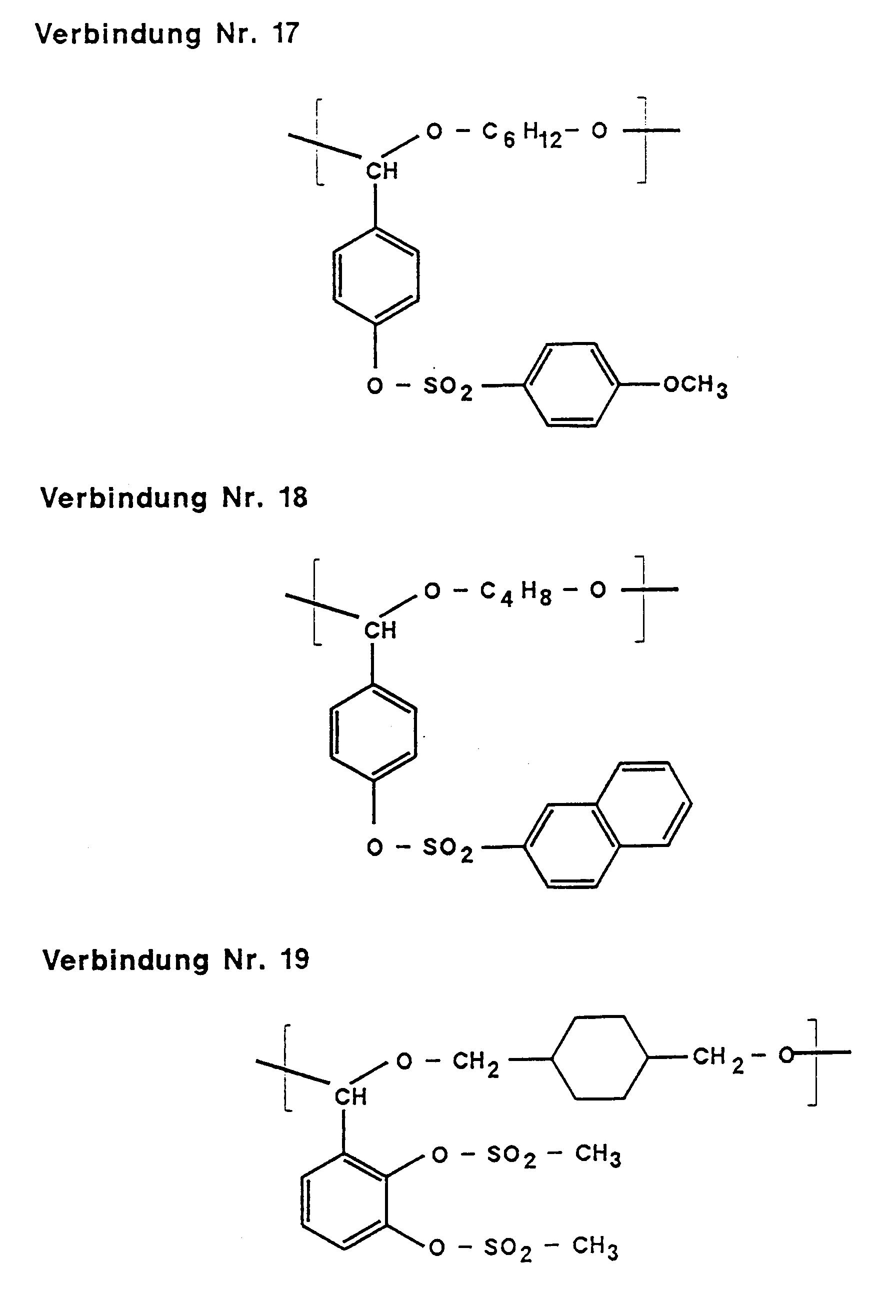 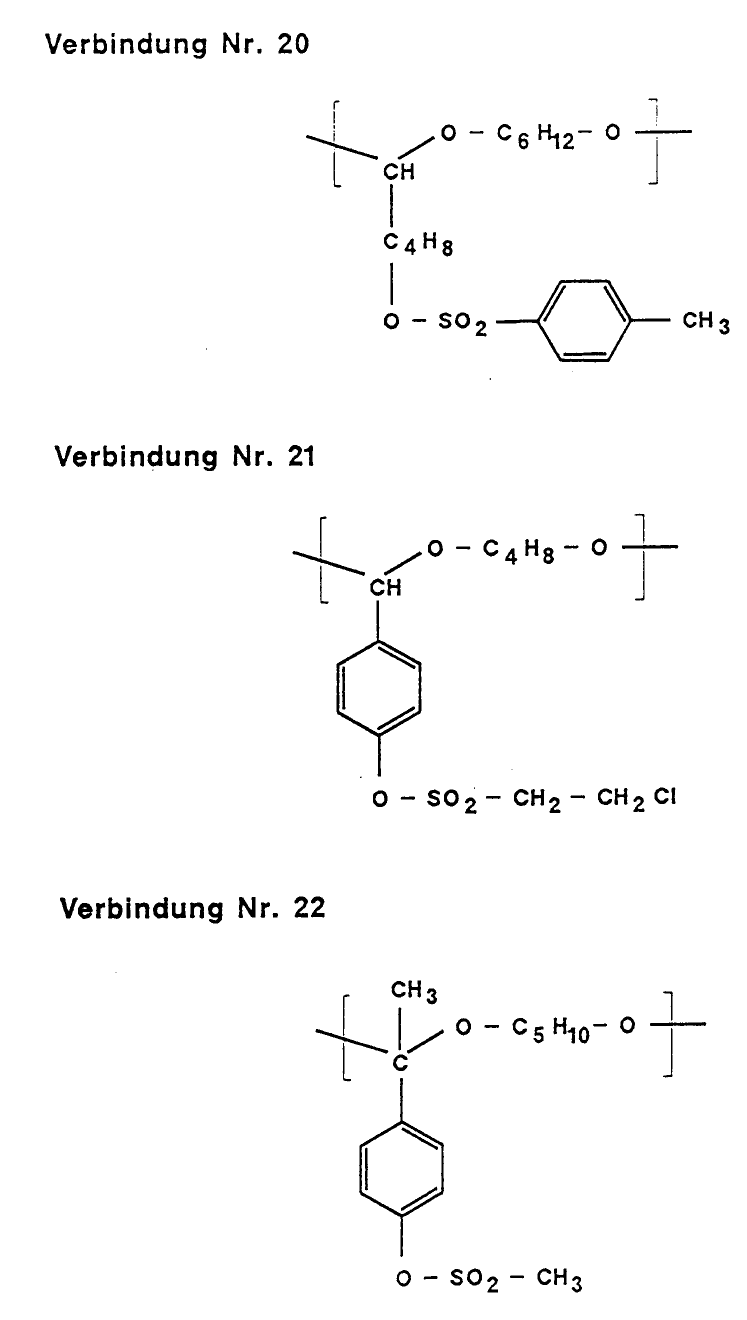 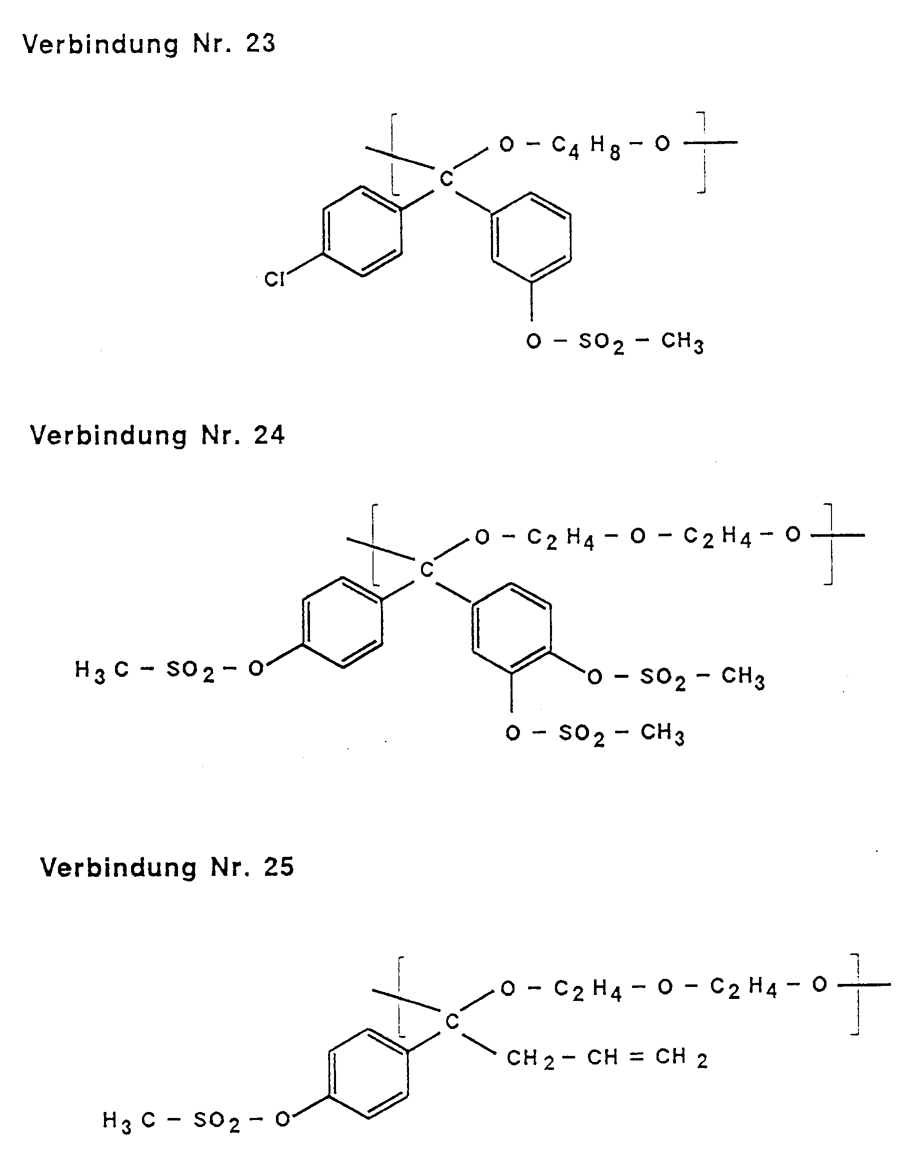 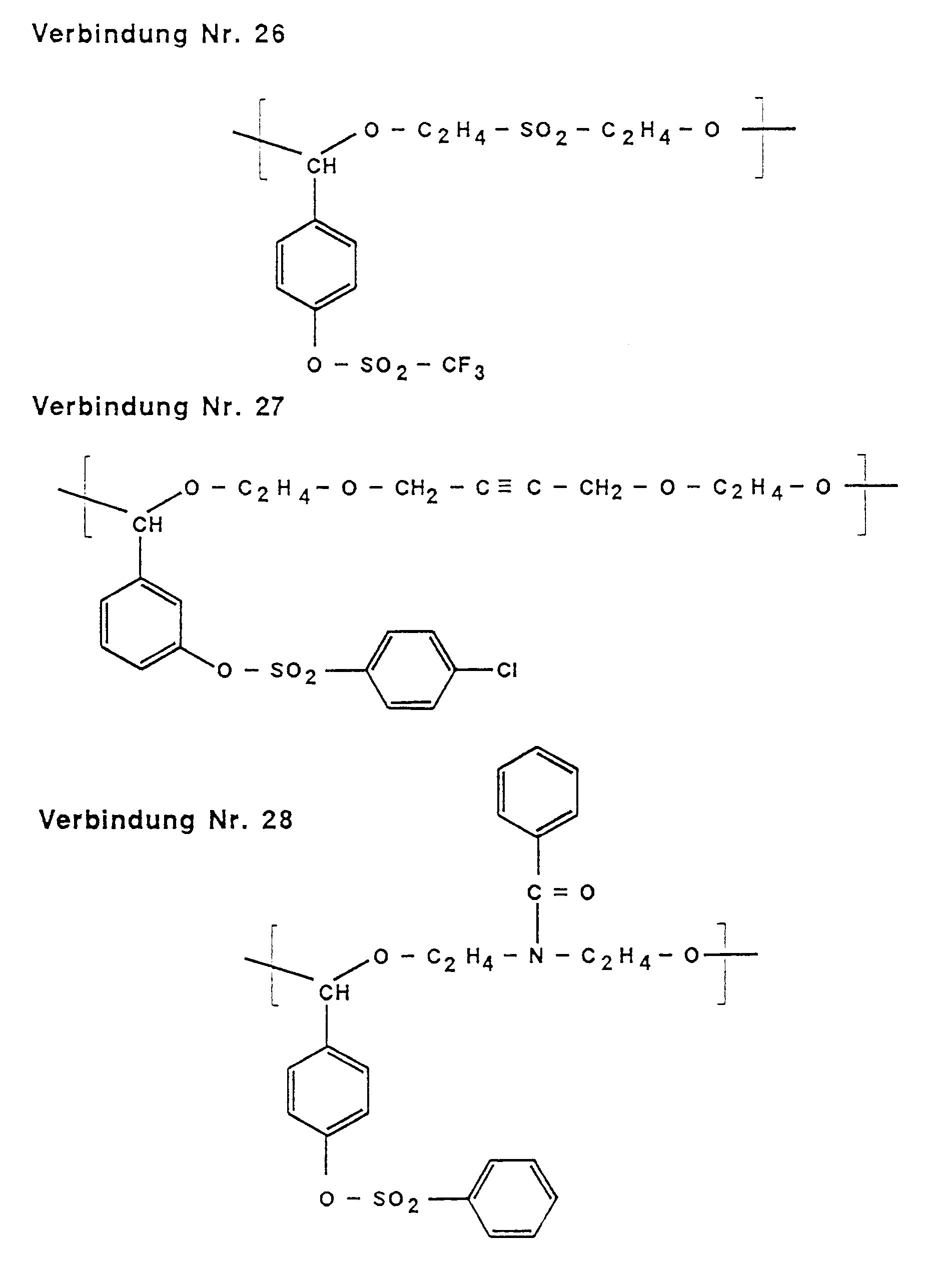 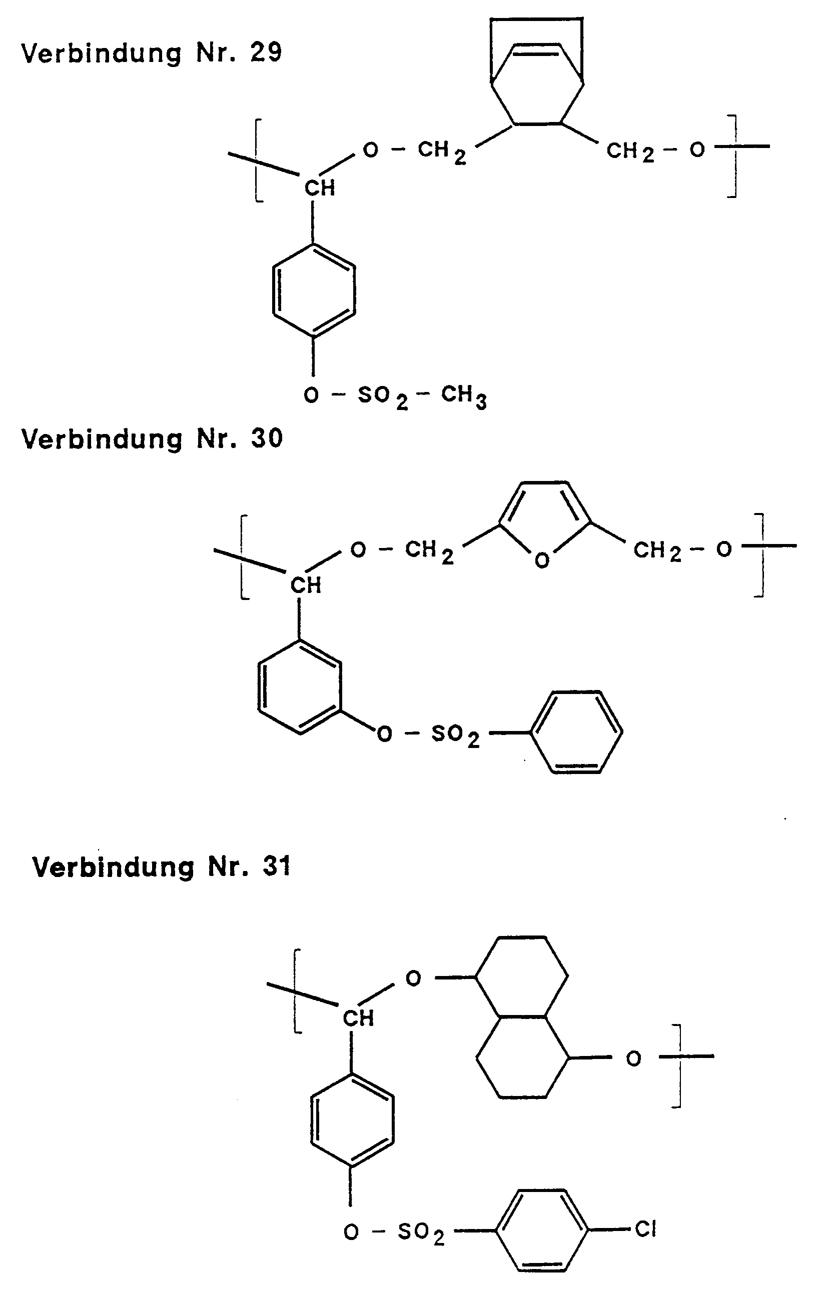 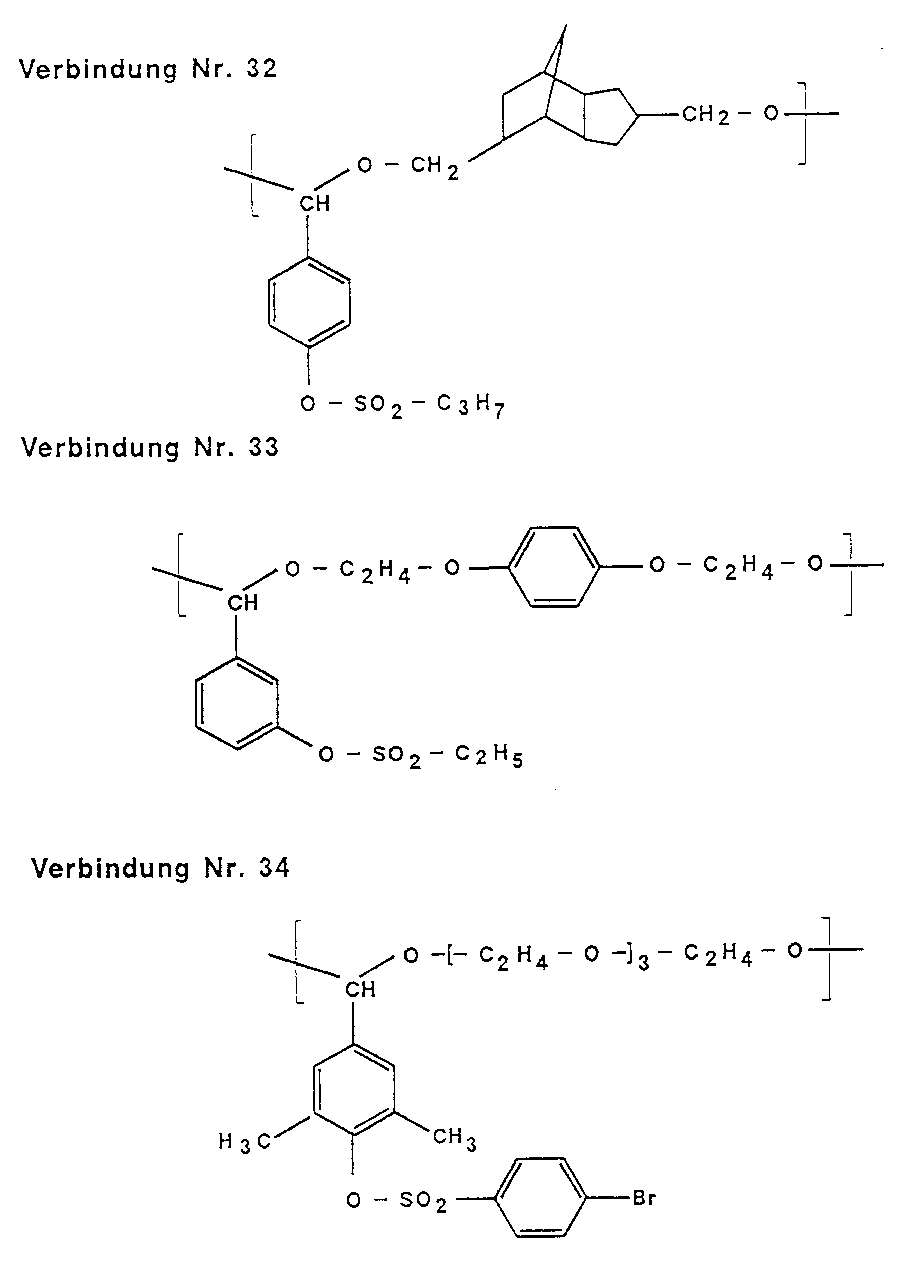 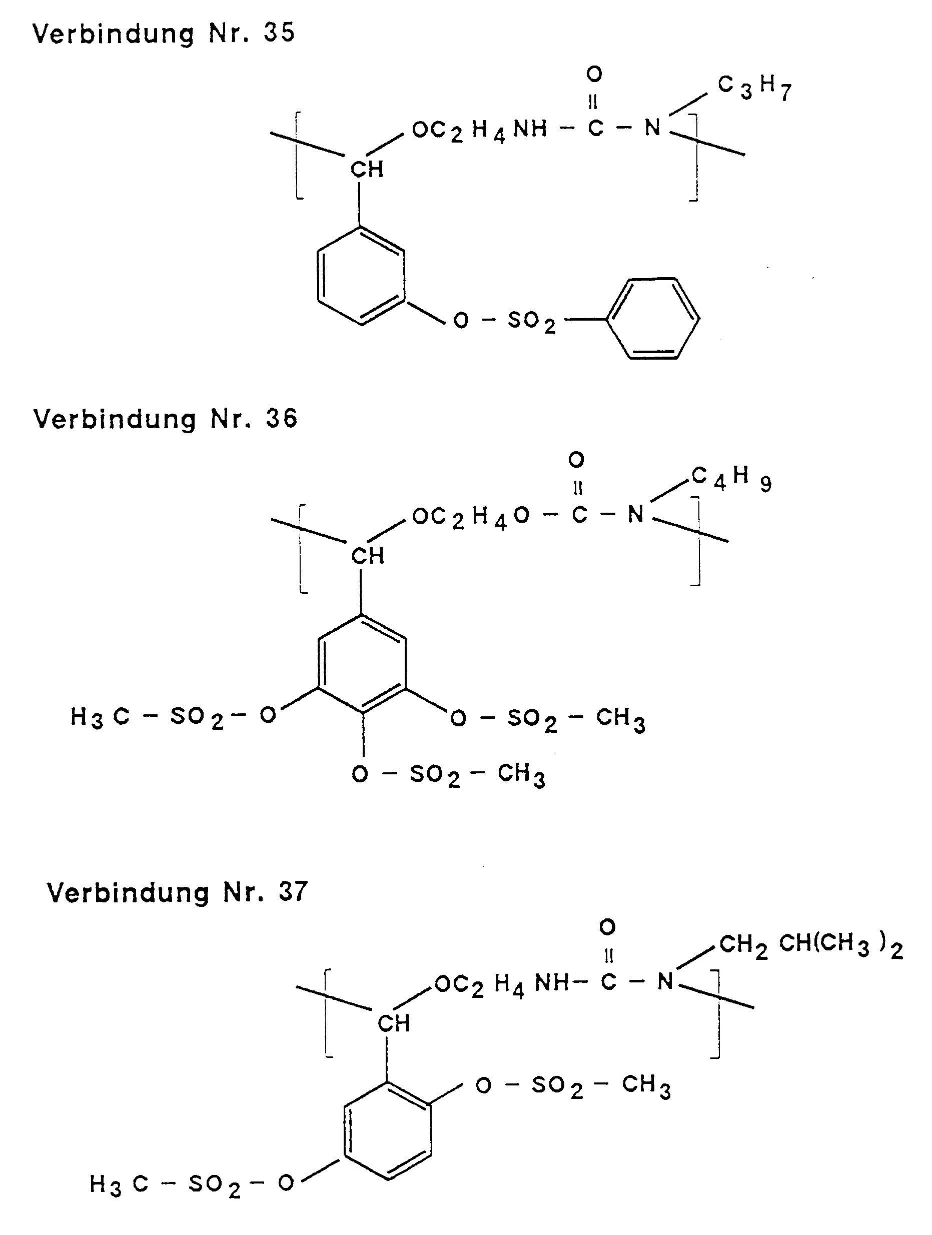 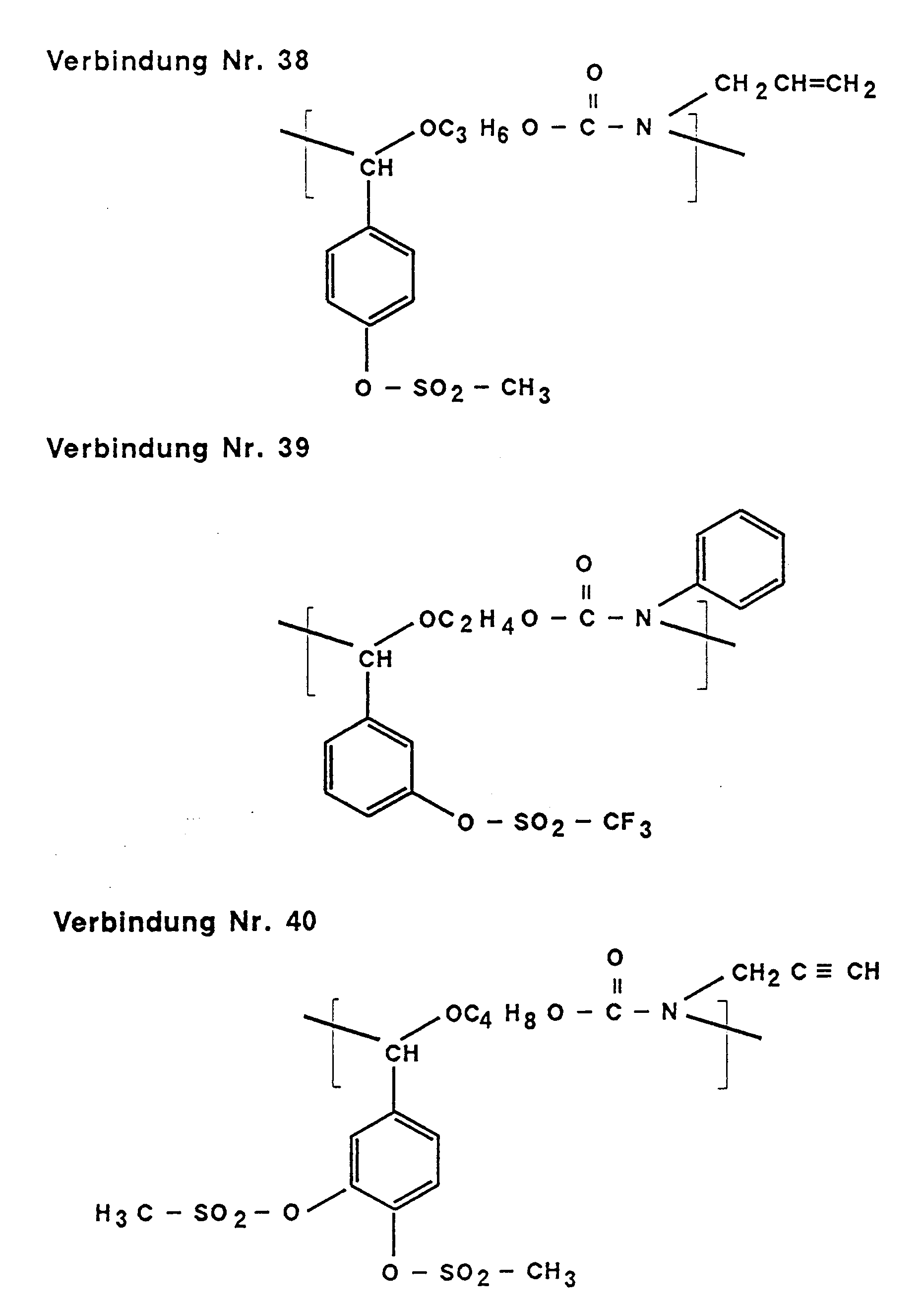 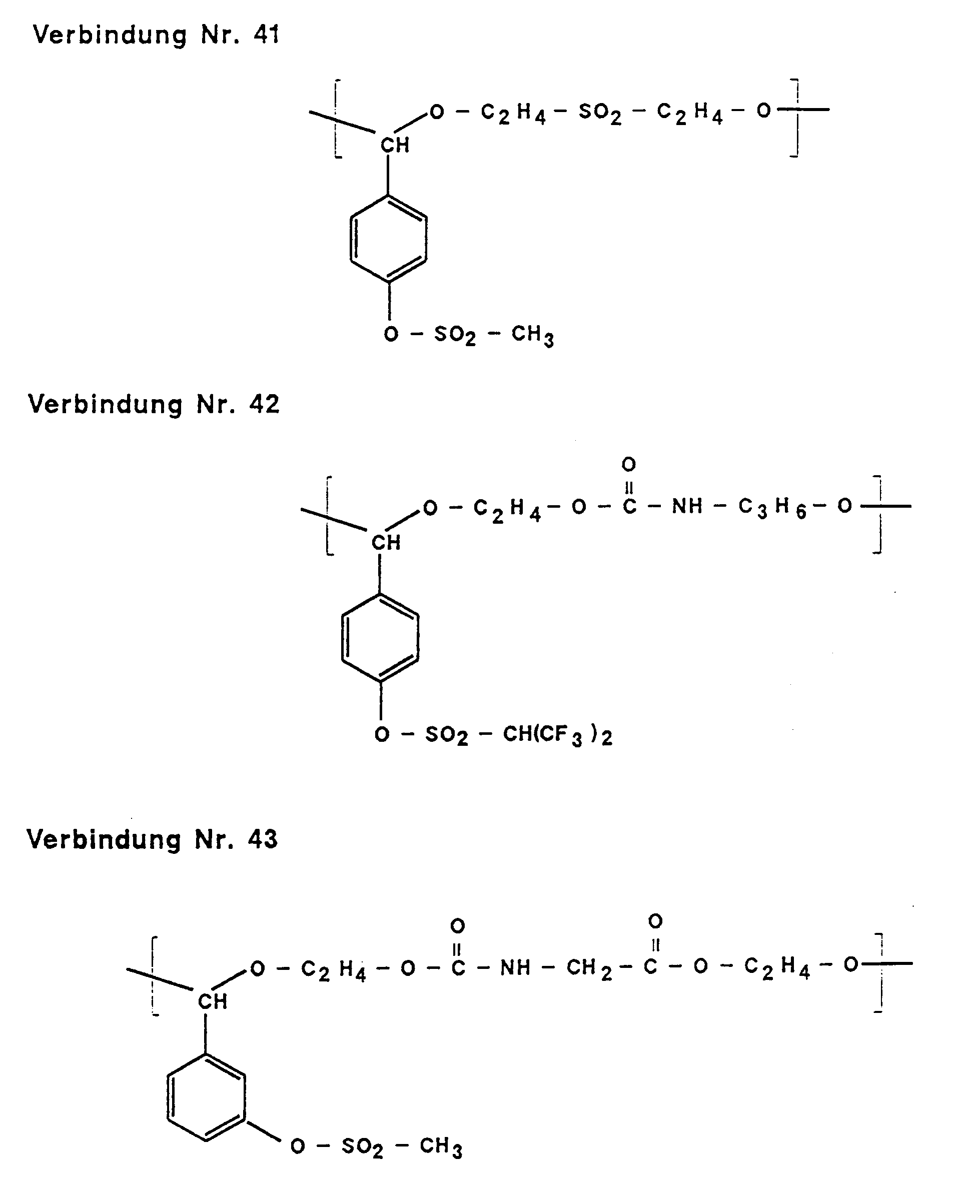 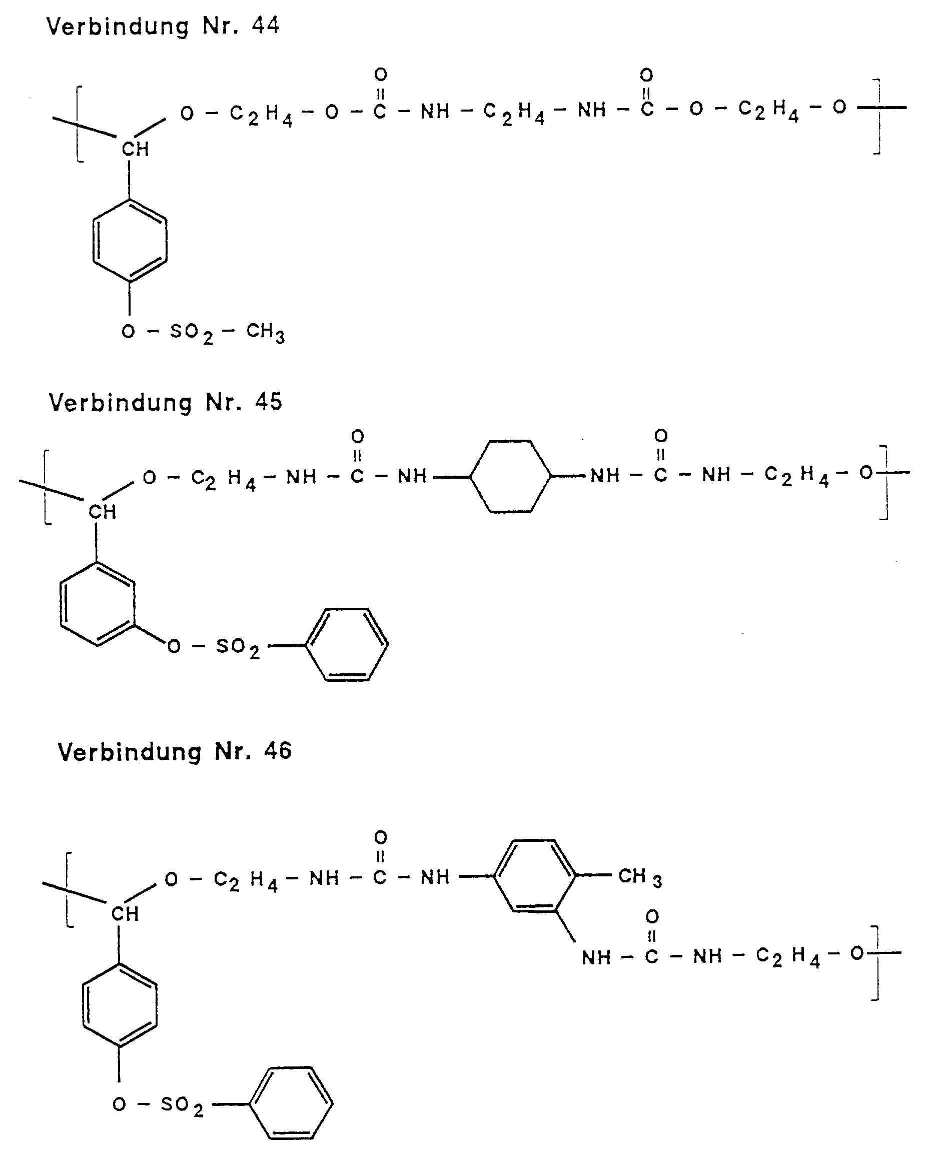 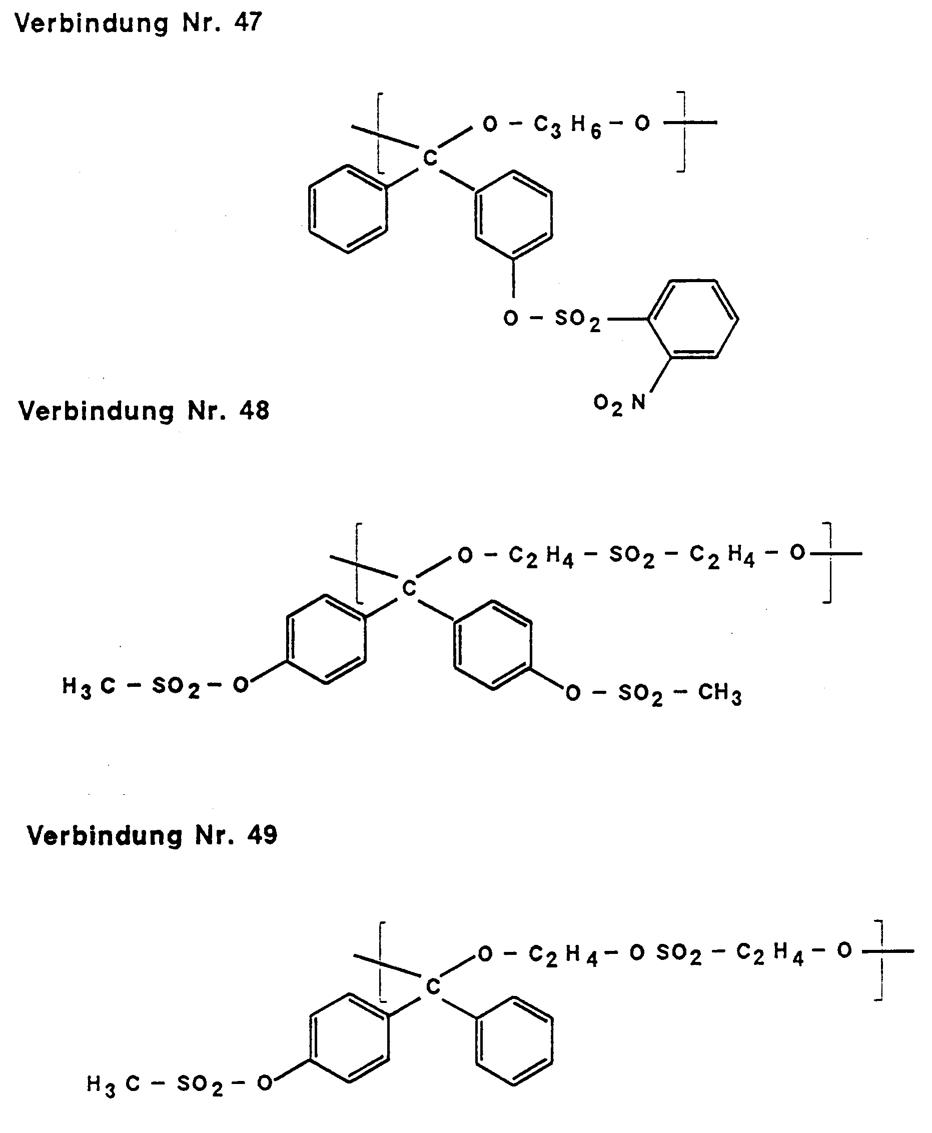
Ihre Syntese erfolgte analog zu literaturbekannten Verfahren. Ihre Authenzität wurde durch Elementaranalyse und ¹H-NMR bestätigt.

Aus der Herstellungsmethodik ist ersichtlich, daß die gebildeten erfindungsgemäßen oligomeren Verbindungen der allgemeinen Formel I durch eine Kondensationsreaktion gebildet werden. Es liegt in der Natur derartiger Reaktionen, daß dabei Produktgemische entstehen, die sich aus einzelnen Oligomeren unterschiedlichen Oligomerisationsgrad zusammensetzen. Durch geeignete Polymeranalytik konnte nachgewiesen werden, daß durch optimierte Reaktionsführung mehr als 90% des Gemischs einen Oligomerisationsgrad zwischen 4 und 40 aufweisen, allerdings lassen sich immer auch monomere, dimere, trimere oder Verbindungen mit einem Oligomerisationsgrad > 40 in geringen Mengen nachweisen. Die Oligomerenzusammensetzung läßt sich jedoch durch Verwendung eindeutig festgelegter Reaktionsbedingungen reproduzierbar gestalten.

Aus der vorstehenden Auswahl erfindungsgemäßer Verbindungen der allgemeinen Formel I geht hervor, daß solche, die Acetalstrukturen aufweisen, besonders bevorzugt sind. Dies ist darauf zurückzuführen, daß ihre Zugänglichkeit sehr gut ist und sie außerdem sowohl in Lösung als auch in Schichten außerordentlich hydrolysestabil sind, und somit ein stabiles Aufzeichnungsmaterial mit diesen Verbindungen hergestellt werden kann. Den erfindungsgemäßen Acetalen liegen, wie in den Herstellungsbeispielen beschrieben, gewisse Aldehyde zugrunde, von denen als Ausgangsverbindungen folgende besonders bevorzugt sind:
2-, 3-, 4-Hydroxybenzaldehyd,
2,3-, 2,4-, 2,5-, 3,4-Dihydroxybenzaldehyd,
2,3,4-, 3,4,5-Trihydroxybenzaldehyd,
4-Hydroxy-3-methyl-benzaldehyd,
2-Hydroxy-4-methoxy-benzaldehyd,
2-Hydroxy-5-methoxy-benzaldehyd,
3-Hydroxy-4-methoxy-benzaldehyd,
4-Hydroxy-3-methoxy-benzaldehyd,
4-Hydroxy-3,5-dimethyl-benzaldehyd,
5-Hydroxy-3,4-dimethoxy-benzaldehyd,
3-Ethoxy-4-hydroxy-benzaldehyd,
2-Hydroxy-5-nitro-benzaldehyd,
3-Hydroxy-4-nitro-benzaldehyd,
4-Hydroxy-3-nitro-benzaldehyd,
5-Hydroxy-2-nitro-benzaldehyd,
2-Hydroxy-naphthalin-1-carbaldehyd.

Diese Aldehyde sind im Handel erhältlich, während sich andere erfindungsgemäß als Vorprodukte geeignete Hydroxyaldehyde nach den unterschiedlichsten Methoden im allgemeinen in einfacher Weise herstellen lassen. Eine Übersicht hierzu ist gegeben in Houben-Weyl, Methoden der organischen Chemie, Bd. 7/1.

Erfindungsgemäß wird ferner ein positiv arbeitendes strahlungsempfindliches Gemisch vorgeschlagen, das als wesentliche Bestandteile
a) ein in Wasser unlösliches, in wäßrig-alkalischen Lösungen lösliches oder zumindest quellbares Bindemittel und
b) eine unter Einwirkung von aktinischer Strahlung eine starke Säure bildende Verbindung mit mindestens einer durch Säure spaltbaren C-O-C-Bindung
enthält, dadurch gekennzeichnet, daß die Verbindung (b) eine Verbindung der vorstehend erläuterten allgemeinen Formel I ist.

Die unter Verwendung der Verbindungen der allgemeinen Formel I erhaltenen erfindungsgemäßen strahlungsempfindlichen Gemische zeichnen sich durch hohe Empfindlichkeiten über einen weiten Spektralbereich aus. Ganz besonders sind sie zur Bestrahlung mit energiereicher UV-Strahlung geeignet, bevorzugt gegenüber Licht einer Wellenlänge von 190 bis 350 nm. Sie zeigen eine hohe thermische Stabilität und schaffen die Möglichkeit, auch feinste Strukturen einer Vorlage strukturgenau wiederzugeben. Durch die Belichtung werden keine korrodierenden Photolyseprodukte gebildet, so daß das Gemisch auch auf empfindlichen Substratmaterialien Verwendung finden kann.

Die im erfindungsgemäßen strahlungsempfindlichen Gemisch enthaltenen säurespaltbaren photolytischen Säurebildner können allein oder in Kombination mit anderen säurelabilen Säurebildnern der erfindungsgemäß genannten Klasse Verwendung finden. Es sind aber auch Kombinationen mit anderen photolytischen Säurebildnern möglich, wozu sich sogar die eingangs erwähnten Onium-Salze, Halogenverbindungen, insbesondere Trichlormethyltriazinderivate oder Trichlormethyloxadiazolderivate, o-Chinondiazidsulfochloride oder Organometall-Organohalogen-Kombinationen eignen. Derartige Kombinationen sind aber insgesamt nicht bevorzugt, da in solchen strahlungsempfindlichen Gemischen unter bestimmten Umständen die schon genannten Nachteile wieder auftreten können.

Der Gehalt an säurelabilen Säurebildnern der allgemeinen Formel I im erfindungsgemäßen Gemisch liegt im allgemeinen zwischen 2 und 60 Gew.-%, bevorzugt bei 5 bis 50 Gew.-%, und besonders bevorzugt bei 10 bis 40 Gew.-%, jeweils bezogen auf das Gesamtgewicht der Schicht.

Den erfindungsgemäßen Gemischen können gegebenenfalls andere säurespaltbare Verbindungen zugefügt werden; dabei haben sich vor allem folgende Verbindungsklassen bewährt:
(1) solche mit mindestens einer Orthocarbonsäureester- und bzw. oder Carbonsäureamidacetalgruppierung, wobei die Verbindungen auch polymeren Charakter haben und die genannten Gruppierungen als verknüpfende Elemente in der Hauptkette oder als seitenständige Substituenten auftreten können (DE-A 26 10 842 und 29 28 636),
(2) Oligomer- oder Polymerverbindungen mit wiederkehrenden Acetal- und/oder Ketalgruppierungen in der Hauptkette (DE-A 23 06 248 und 27 18 254),
(3) Verbindungen mit mindestens einer Enolether- oder N-Acyliminocarbonatgruppierung (EP-A 0 006 626 und 0 006 627),
(4) cyclische Acetale oder Ketale von β-Ketoestern oder -amiden (EP-A 0 202 196),
(5) Verbindungen mit Silylethergruppierungen (DE-A 35 44 165 und 36 01 264),
(6) Verbindungen mit Silyl-enolethergruppierungen (DE-A 37 30 785 und 37 30 783),
(7) Monoacetale bzw. Monoketale, deren Aldehyd- bzw. Ketonkomponente eine Löslichkeit im Entwickler zwischen 0,1 und 100 g/l aufweisen (DE-A 37 30 787),
(8) Ether auf der Basis tertiärer Alkohole (US-A 4,603,101) und
(9) Carbonsäureester und Carbonate tertiärer, allylischer oder benzylischer Alkohole [US-A 4,491,628 und J. M. Fréchet et al., J. Imaging Sci. 30, 59-64 (1986)].

Es können auch Mischungen der genannten säurespaltbaren Verbindungen eingesetzt werden. Unter diesen bevorzugt verwendbar sind allerdings säurespaltbare Verbindungen, die einem der obengenannten Typen (1) bis (9) zuzuordnen sind, und darunter besonders diejenigen, die eine durch Säure spaltbare C-O-C-Bindung aufweisen. Unter diesen besonders bevorzugt sind diejenigen Verbindungen, die den Typen (1), (2), (7) und (9) angehören. Unter Typ (2) sind besonders die polymeren Acetale hervorzuheben; von den säurespaltbaren Verbindungen des Typs (7) insbesondere diejenigen, deren Aldehyd- bzw. Ketonkomponente einen Siedepunkt oberhalb von 150 °C, vorzugsweise oberhalb von 200 °C, aufweist. Insgesamt jedoch sind solche Mischungen nicht bevorzugt.

Die Verbindung (b) bzw. die Kombination von Verbindungen (b) ist in einer Konzentration von 2 bis 60 Gew.-% enthalten.

Das erfindungsgemäße strahlungsempfindliche Gemisch enthält ferner mindestens ein polymeres, in Wasser unlösliches, in wäßrig-alkalischen Lösungen dagegen lösliches, zumindest quellbares Bindemittel. Das Bindemittel zeichnet sich im besonderen dadurch aus, daß es mit den übrigen Bestandteilen des erfindungsgemäßen strahlungsempfindlichen Gemisches gut verträglich ist und insbesondere im Wellenlängenbereich von 190 bis 350 nm eine möglichst geringe Eigenabsorption, d.h. eine hohe Transparenz, aufweist. Bindemittel auf der Basis von Novolak-Kondensationsharzen, die in der Regel in Kombination mit Naphthochinondiaziden als photoaktive Komponenten eingesetzt werden, erfüllen diese Bedingung nicht. Zwar lassen Novolak-Kondensationsharze nach bildmäßiger Belichtung in den belichteten Bereichen eine Erhöhung der Löslichkeit gegenüber wäßrig-alkalischen Entwicklern erkennen, doch ist ihre Eigenabsorption im Bereich der für die Bestrahlung gewünschten kurzen Wellenlänge unerwünscht hoch.

Novolak-Kondensationsharze können aber in Mischung mit anderen, als Bindemittel geeigneten Harzen mit höherer Transparenz eingesetzt werden. Die Mischungsverhältnisse richten sich dabei vorwiegend nach der Art des mit dem Novolakharz zu mischenden Bindemittels. Insbesondere spielen dessen Grad an Eigenabsorption im genannten Wellenlängenbereich, aber auch die Mischbarkeit mit den anderen Bestandteilen des strahlungsempfindlichen Gemisches eine entscheidende Rolle. Im allgemeinen kann aber das Bindemittel des erfindungsgemäßen strahlungsempfindlichen Gemisches bis zu 30 Gew.-%, insbesondere bis zu 20 Gew.-%, eines Novolak-Kondensationsharzes enthalten.

Als Bindemittel geeignet sind Homo- oder Copolymere des p-Hydroxystyrols sowie seiner Alkylderivate, z. B. des 3-Methyl-4-hydroxystyrols, des 3,5-Dimethyl-4-hydroxystyrols oder des 2,3-Dimethyl-4-hydroxystyrols sowie Homo- oder Copolymere anderer Polyvinylphenole, z. B. des 2- oder 3-Hydroxystyrols, oder des 4-Methyl-3-hydroxystyrols oder die Ester der (Meth)acrylsäure mit Phenolen, z. B. Brenzkatechin, Resorcin, Hydrochinon, Pyrogallol oder Aminophenole sowie die entsprechenden Amide mit aromatischen Aminen. Als Comonomere können polymerisierbare Verbindungen wie Styrol, Methylmethacrylat, Methylacrylat oder ähnliche eingesetzt werden.

Gemische mit erhöhter Plasmabeständigkeit werden dann erhalten, wenn zur Herstellung von Copolymeren des obigen Typs Silicium enthaltende Vinylmonomere, z. B. Vinyltrimethylsilan oder Allyltrimethylsilan, verwendet werden. Die Transparenz dieser Bindemittel ist im interessierenden Bereich im allgemeinen höher, so daß eine verbesserte Strukturierung möglich ist.

Mit gleichem Erfolg lassen sich auch Homo- oder Copolymere des Maleinimids verwenden. Auch diese Bindemittel zeigen hohe Transparenz im beschriebenen Wellenlängenbereich. Als Comonomere werden auch hier bevorzugt Styrol, substituierte Styrole, Vinylphenole, Vinylether, Vinylester, Vinylsilylverbindungen oder (Meth)acrylsäureester eingesetzt.

Schließlich sind darüber hinaus auch Copolymere des Styrols mit Comonomeren verwendbar, die in wäßrig alkalischen Lösungen eine Löslichkeitserhöhung bewirken. Hierunter zählen beispielsweise Maleinsäureanhydrid, Maleinsäurehalbester oder dergleichen.

Die genannten Bindemittel können auch untereinander gemischt werden, sofern sich dadurch die optische Qualität des strahlungsempfindlichen Gemisches nicht verschlechtert. Bindemittelgemische sind jedoch nicht bevorzugt.

Die Menge des Bindemittels beträgt im allgemeinen 40 bis 98 Gew.-%, insbesondere 50 bis 95 Gew.-%, vorzugsweise 60 bis 90 Gew.-%, bezogen auf das Gesamtgewicht der strahlungsempfindlichen Mischung.

Die Extinktion des Bindemittels bzw. der Kombination von Bindemitteln (a) im Wellenlängenbereich der Empfindlichkeit der Verbindung (b) sollte weniger als 0,5 »m⁻¹ betragen.

Ferner können den erfindungsgemäßen strahlungsempfindlichen Gemischen gegebenenfalls Farbstoffe, Pigmente, Weichmacher, Netzmittel und Verlaufmittel, aber auch Polyglykole, Celluloseether, z. B. Ethylcellulose, zur Verbesserung spezieller Erfordernisse, wie Flexibilität, Haftung und Glanz, zugesetzt werden.

Soll ein Substrat beschichtet werden, so wird das erfindungsgemäße strahlungsempfindliche Gemisch zweckmäßig in einem Lösemittel oder in einer Kombination von Lösemitteln gelöst. Hierfür besonders geeignet sind Ethylenglykol, Ethylenglykolmonomethylether, Ethylenglykoldimethylether, Ethylenglykolmonoethylether oder Propylenglykolmonoalkylether, insbesondere Propylenglykolmethylether, aliphatische Ester (z. B. Ethylacetat, Hydroxyethylacetat, Alkoxyethylacetat, n-Butylacetat, Propylenglykolalkyletheracetat, insbesondere Propylenglykolmethyletheracetat oder Amylacetat), Ether (z. B. Dioxan), Ketone (z. B. Methylethylketon, Methylisobutylketon, Cyclopentanon und Cyclohexanon), Dimethylformamid, Dimethylacetamid, Hexamethylphosphorsäureamid, N-Methyl-pyrrolidon, Butyrolacton, Tetrahydrofuran und Mischungen derselben] gelöst. Besonders bevorzugt werden Glykolether, aliphatische Ester sowie Ketone.

Letztendlich hängt die Wahl der Lösemittel von dem angewandten Beschichtungsverfahren, der gewünschten Schichtstärke und den Trocknungsbedingungen ab. Ebenso müssen die Lösemittel chemisch neutral sein, d. h. sie dürfen nicht mit den übrigen Schichtkomponenten irreversibel reagieren.

Die mit den genannten Lösemitteln hergestellte Lösung hat in der Regel einen Feststoffgehalt von 5 bis 60 Gew.-%, vorzugsweise bis 50 Gew.-%.

Gegenstand der Erfindung ist schließlich auch ein strahlungsempfindliches Aufzeichnungsmaterial, das im wesentlichen aus einem Substrat und einer darauf befindlichen strahlungsempfindlichen Schicht aus dem erfindungsgemäßen strahlungsempfindlichen Gemisch besteht.

Als Substrate kommen alle Materialien in Frage, aus denen Kondensatoren, Halbleiter, mehrlagige gedruckte Schaltungen oder integrierte Schaltkreise bestehen bzw. hergestellt werden können. Speziell sind Siliciumsubstrate zu nennen, die auch thermisch oxidiert und/oder mit Aluminium beschichtet, aber auch dotiert sein können. Daneben sind alle anderen in der Halbleitertechnologie üblichen Substrate möglich, wie Siliciumnitrid, Galliumarsenid und Indiumphosphid. Weiterhin kommen die aus der Flüssigkristalldisplay-Herstellung bekannten Substrate in Frage, wie z. B. Glas und Indium-Zinnoxid, ferner Metallplatten und -folien - beispielsweise aus Aluminium, Kupfer, Zink-, Bimetall- und Trimetallfolien, aber auch elektrisch nichtleitende Folien, die mit Metallen bedampft sind, und Papier. Diese Substrate können thermisch vorbehandelt, oberflächlich aufgerauht, angeätzt oder, zur Verbesserung erwünschter Eigenschaften, z. B. zur Erhöhung der Hydrophilie, mit Chemikalien vorbehandelt sein.

Um der strahlungsempfindlichen Schicht einen besseren Zusammenhalt und/oder eine bessere Haftung auf der Substratoberfläche zu verleihen, kann in ihr ein Haftvermittler enthalten sein. Bei Silicium- bzw. Siliciumdioxid-Substraten kommen hierfür Haftvermittler vom Aminosilan-Typ, z. B. 3-Aminopropyltriethoxysilan oder Hexamethyldisilazan, in Frage.

Geeignete Träger für die Herstellung von photomechanischen Aufzeichnungsschichten, wie Druckformen für den Hochdruck, Flachdruck, Siebdruck und Flexodruck sind besonders Aluminiumplatten, die gegebenenfalls vorher anodisch oxidiert, gekörnt und/oder silikatisiert werden, daneben Zink- und Stahlplatten, die gegebenenfalls verchromt werden, sowie Kunststoffolien und Papier.

Das erfindungsgemäße Aufzeichnungsmaterial wird mit aktinischer Strahlung bildmäßig belichtet. Als Strahlungsquellen eignen sich besonders Metallhalogenidlampen, Kohlebogenlampen, Xenonlampen und Quecksilberdampflampen. Ebenso kann eine Belichtung mit energiereicher Strahlung wie Laser-, Elektronen- oder Röntgenstrahlung erfolgen. Besonders bevorzugt sind jedoch Lampen, die Licht einer Wellenlänge von 190 bis 260 nm ausstrahlen können, d.h. insbesondere Xenon- und Quecksilberdampflampen. Darüber hinaus lassen sich auch Laserlichtquellen verwenden, z. B. Excimerlaser, insbesondere KrF- oder ArF-Laser, die bei 248 bzw. 193 nm emittieren. Die Strahlungsquellen müssen in den genannten Wellenlängenbereichen eine ausreichende Emission aufweisen.

Die Stärke der lichtempfindlichen Schicht hängt vom Verwendungszweck ab. Sie beträgt im allgemeinen zwischen 0,1 und 100 »m, bevorzugt zwischen 1 und 10 »m.

Gegenstand der Erfindung ist ferner ein Verfahren zur Herstellung eines strahlungsempfindlichen Aufzeichnungsmaterials. Das Auftragen des strahlungsempfindlichen Gemisches auf das Substrat kann durch Aufsprühen, Fließbeschichten, Walzen, Schleuder- und Tauchbeschichten erfolgen. Danach wird das Lösemittel durch Verdampfen entfernt, so daß auf der Oberfläche des Substrats die strahlungsempfindliche Schicht zurückbleibt. Die Entfernung des Lösemittels kann durch Erhitzen der Schicht auf Temperaturen bis zu 150 °C gefördert werden. Das Gemisch kann aber auch zunächst auf obengenannte Weise auf einen Zwischenträger aufgetragen werden, von dem aus es unter Druck und erhöhter Temperatur auf das endgültige Trägermaterial übertragen wird. Als Zwischenträger können grundsätzlich alle auch als Trägermaterialien geeigneten Materialien Anwendung finden. Anschließend wird die Schicht bildmäßig bestrahlt und zur Verstärkung der Spaltreaktion erwärmt Danach behandelt man die Schicht mit einer Entwicklerlösung, die die bestrahlten Bereiche der Schicht löst und entfernt, so daß ein Abbild der bei der bildmäßigen Bestrahlung verwendeten Vorlage auf der Substratoberfläche verbleibt.

Als Entwickler eignen sich besonders wäßrige Lösungen, die Silikate, Metasilikate, Hydroxide, Hydrogen- und Dihydrogenphosphate, Carbonate oder Hydrogencarbonate von Alkalimetall-, Erdalkalimetall- und/oder Ammoniumionen enthalten, aber auch Ammoniak und dergleichen. Metallionenfreie Entwickler werden in der US-A 4 729 941, der EP-A 0 062 733, der US-A 4 628 023, der US-A 4 141 733, der EP-A 0 097 282 und der EP-A 0 023 758 beschrieben. Der Gehalt dieser Substanzen in der Entwicklerlösung beträgt im allgemeinen 0,1 bis 15 Gew.-%, vorzugweise 0,5 bis 5 Gew.-%, bezogen auf das Gewicht der Entwicklerlösung. Metallionenfreie Entwickler werden bevorzugt verwendet. Den Entwicklern können gegebenenfalls geringe Mengen eines Netzmittels zugesetzt sein, um die Ablösung der löslichen Bereiche der Schicht zu erleichtern.

Die entwickelten Schichtstrukturen können nachgehärtet werden. Dies geschieht im allgemeinen durch Erhitzen auf einer hot-plate bis zu einer Temperatur unterhalb der Fließtemperatur und anschließendes ganzflächiges Belichten mit dem UV-Licht einer Xenon-Quecksilber-Dampflampe (Bereich von 200 bis 250 nm). Durch die Nachhärtung werden die Bildstrukturen vernetzt, so daß sie im allgemeinen eine Fließbeständigkeit bis zu Temperaturen von über 200 °C aufweisen. Die Nachhärtung kann auch ohne Temperaturerhöhung allein durch Bestrahlen mit energiereichem UV-Licht erfolgen.

Verwendung findet das erfindungsgemäße strahlungsempfindliche Gemisch bei der Herstellung von integrierten Schaltungen oder von diskreten elektronischen Bausteinen mit lithographischen Prozessen, da sie eine hohe Lichtempfindlichkeit aufweisen, besonders bei Bestrahlung mit Licht einer Wellenlänge zwischen 190 bis 350 nm. Da die Gemische bei Belichtung sehr gut ausbleichen, lassen sich feinere Strukturen erzielen als dies mit den bekannten Gemischen möglich ist. Die entwickelte Resistschicht dient dabei als Maske für die folgenden Prozeßschritte. Solche Schritte sind z. B. das Ätzen des Schichtträgers, das Implantieren von Ionen in den Schichtträger oder das Abscheiden von Metallen oder anderen Materialien auf dem Schichtträger.

Die folgenden Beispiele 1 bis 10 belegen die Eignung des erfindungsgemäßen Gemisches für Aufzeichnungsmaterialien in der Mikrolithographie unter Verwendung von energiereicher Strahlung. Anhand der Vergleichsbeispiele 11 und 12 wird die Überlegenheit der erfindungsgemäßen Gemische gegenüber dem Stand der Technik belegt.

In den Beispielen sind die Mengen in der Regel als Gewichtsteile (Gt) angegeben. Wenn nichts anderes angegeben ist, sind Prozentzahlen und Mengenverhältnisse in Gewichtseinheiten zu verstehen.

### Beispiel 1

Es wurde eine Beschichtungslösung hergestellt aus
6,0 Gt eines Copolymeren aus Styrol/p-Hydroxystyrol (Molverhältnis 30:70) mit einem mittleren Molekulargewicht von 27.000,
1,5 Gt eines Kresol-Formaldehyd-Novolaks mit einem Erweichungsbereich von 105 bis 120 °C und
2,0 Gt der Verbindung 1 in
42 Gt Propylenglykol-monomethylether-acetat.

Die Lösung wurde durch ein Filter mit einem Porendurchmesser von 0,2 »m filtriert und auf einen mit einem Haftvermittler (Hexamethyldisilazan) behandelten Wafer bei 3.300 Umdrehungen aufgeschleudert. Nach 1 Minute Trocknen bei 100 °C auf der hot plate wurde eine Schichtdicke von 1,05 »m erhalten.

Das Aufzeichnungsmaterial wurde bildmäßig unter einer Vorlage mit der UV-Strahlung einer Xenon-Quecksilberdampflampe (Filter mit Transmission von 240 bis 260 nm) mit einer Energie von 78 mJ/cm² belichtet und vor der Entwicklung etwa 30 Minuten bei Raumtemperatur gelagert.

Entwickelt wurde das Aufzeichnungsmaterial mit einem 0,3 n alkalischen Entwickler folgender Zusammensetzung:
5,3 Gt Natriummetasilikat x 9 H₂O,
3,4 Gt Trinatriumphosphat x 12 H₂O,
0,3 Gt Natriumdihydrogenphosphat und
191 Gt vollentsalztes Wasser.

Nach einer Entwicklungsdauer von 60 s erhielt man ein fehlerfreies Abbild der Maske mit steilen Resistflanken, wobei auch Strukturen < 0,6 »m detailgetreu aufgelöst waren. Eine Untersuchung der Flanken der Resistprofile mittels Rasterelektronenmikroskopie belegte, daß diese praktisch senkrecht zur Substratoberfläche ausgerichtet waren.

### Beispiel 2

Es wurde eine Beschichtungslösung hergestellt aus
7,5 Gt eines Copolymeren aus Styrol/p-Hydroxystyrol (Molverhältnis 20:80) mit einem mittleren Molekulargewicht von 32.000 und
2,0 Gt der Verbindung 2 in
42 Gt Propylenglykolmonomethyletheracetat.

Die Lösung wurde durch ein Filter mit einem Porendurchmesser von 0,2 »m filtriert und auf einen mit einem Haftvermittler (Hexamethyldisilazan) behandelten Wafer bei 3.300 Umdrehungen aufgeschleudert. Nach 1 Minute Trocknen bei 100 °C auf der hot plate wurde eine Schichtdicke von 1,12 »m erhalten.

Das Aufzeichnungsmaterial wurde bildmäßig unter einer Vorlage mit der UV-Strahlung einer Xenon-Quecksilberdampflampe bei 240 bis 260 nm mit einer Energie von 82 mJ/cm² belichtet, etwa 30 Minuten bei Raumtemperatur gelagert und anschließend mit dem in Beispiel 1 beschriebenen Entwickler verarbeitet.

Nach einer Entwicklungsdauer von 60 s erhielt man ein fehlerfreies Abbild der Maske mit hoher Flankenstabilität, wobei auch hier Strukturen < 0,6 »m detailgetreu aufgelöst waren.

### Beispiel 3

Ein entsprechend Beispiel 1 hergestellter Wafer wurde unter einer Vorlage mit KrF-Excimer-Laser-Strahlung einer Wellenlänge von 248 nm mit einer Energie von 100 mJ/cm² bestrahlt. Nach der Entwicklung wurde ein originalgetreues Abbild der Vorlage erhalten.

### Beispiel 4

Es wurde eine Beschichtungslösung hergestellt aus
7,5 Gt des in Beispiel 2 angegebenen Copolymeren und
2,0 Gt der Verbindung 3 in
42 Gt Propylenglykol-monomethylether-acetat.

Die Lösung wurde durch ein Filter mit einem Porendurchmesser von 0,2 »m filtriert und auf insgesamt zwei mit einem Haftvermittler (Hexamethyldisilazan) behandelte Wafer bei 3.300 Umdrehungen aufgeschleudert. Nach 1 Minute Trocknen bei 100 °C auf der hot plate wurde in beiden Fällen eine Schichtdicke von 1,04 »m erhalten.

Einer der beschichteten Wafer wurde bildmäßig unter einer Vorlage mit der UV-Strahlung einer Xenon-Quecksilberdampflampe bei 240 bis 260 nm mit einer Energie von 60 mJ/cm² belichtet, 75 s auf 100 °C erwärmt und anschließend mit einem Entwickler verarbeitet, der aus 3 % Tetramethylammoniumhydroxid und 97 % entionisiertem Wasser bestand.

Nach einer Entwicklungsdauer von 60 s erhielt man ein fehlerfreies Abbild der Maske mit hoher Flankenstabilität, wobei auch hier Strukturen < 0,6 »m detailgetreu aufgelöst waren.

Der zweite Wafer wurde nach 14 Tagen wie oben beschrieben belichtet, getempert und entwickelt. Es wurden praktisch die gleichen Ergebnisse erzielt wie vorstehend beschrieben. Dies bedeutet, daß das Gemisch in getrockneter Form auf einem Substrat aufgetragen eine ausgezeichnete Stabilität aufweist.

### Beispiel 5

Es wurde eine Beschichtungslösung hergestellt aus
7,5 Gt eines Homopolymeren aus 3-Methyl-4-hydroxystyrol mit einem mittleren Molekulargewicht von 25.000 und
2,0 Gt der Verbindung 1 in
42 Gt Propylenglykol-monomethylether-acetat.

Die Lösung wurde durch ein Filter mit einem Porendurchmesser von 0,2 »m filtriert, und in zwei gleiche Teile getrennt. Der eine Teil wurde auf einen mit einem Haftvermittler (Hexamethyldisilazan) behandelten Wafer bei 3.300 Umdrehungen aufgeschleudert. Nach 1 Minute Trocknen bei 100 °C wurde eine Schichtdicke von 1,04 »m erhalten.

Das Aufzeichnungsmaterial wurde bildmäßig unter einer Vorlage mit der Strahlung einer Xenon-Quecksilberdampflampe bei 240 bis 260 nm mit einer Energie von 80 mJ/cm² belichtet, 75 s bei 100 °C erwärmt und anschließend mit dem in Beispiel 4 beschriebenen Entwickler verarbeitet.

Nach einer Entwicklungsdauer von 60 s erhielt man ein fehlerfreies Abbild der Maske mit hoher Flankenstabilität, wobei auch hier Strukturen < 0,6 »m detailgetreu aufgelöst waren.

Der zweite Teil wurde nach 20 Wochen Lagerung im Kühlschrank der gleichen Prozedur unterzogen. Es wurden identische Ergebnisse erhalten, woraus hervorgeht, daß das Gemisch in Lösung außerordentlich gute Stabilität aufweist.

### Beispiel 6

Es wurde eine Beschichtungslösung hergestellt aus
7,5 Gt eines Copolymeren aus 3,5-Dimethyl-4-hydroxystyrol/4-Hydroxystyrol (Molverhältnis 20:80) mit dem mittleren Molekulargewicht 25.000 und
2,0 Gt der Verbindung 4 in
42 Gt Propylenglykol-monomethylether-acetat.

Die Lösung wurde durch ein Filter mit einem Porendurchmesser von 0,2 »m filtriert und auf einen mit einem Haftvermittler (Hexamethyldisilazan) behandelten Wafer bei 3.300 Umdrehungen aufgeschleudert. Nach 1 Minute Trocknen bei 100 °C wurde eine Schichtdicke von 1,08 »m erhalten.

Das Aufzeichnungsmaterial wurde bildmäßig unter einer Vorlage mit der UV-Strahlung einer Xenon-Quecksilberdampflampe bei 240 bis 260 nm mit einer Energie von 72 mJ/cm² belichtet, 75 Sekunden auf 100 °C erwärmt, und anschließend mit dem in Beispiel 1 beschriebenen Entwickler verarbeitet.

Nach einer Entwicklungsdauer von 60 s erhielt man ein fehlerfreies Abbild der Maske mit hoher Flankenstabilität, wobei auch hier Strukturen < 0,6 »m detailgetreu aufgelöst waren.

### Beispiel 7

Beispiel 4 wurde zweimal wiederholt, mit der Abweichung, daß nicht 60 s lang bei 100 °C getrocknet wurde, sondern 90 s lang bei 90 °C bzw. 60 s lang bei 110 In beiden Fällen waren die Ergebnisse praktisch gleich und stimmten mit den im Beispiel 4 beschriebenen überein. Das bedeutet, daß das erfindungsgemäße Aufzeichnungsmaterial einen großen Verarbeitungsspielraum aufweist.

### Beispiel 8

Es wurde eine Beschichtungslösung hergestellt aus
7,5 Gt eines Copolymeren aus Styrol und Maleimid (Molverhältnis 1:1) mit einem Erweichungsbereich von 165 bis 180 °C und
2,0 Gt der Verbindung 4 in
42 Gt Cyclohexanon.

Die Lösung wurde durch ein Filter mit einem Porendurchmesser von 0,2 »m filtriert und auf einen mit einem Haftvermittler (Hexamethyldisilazan) behandelten Wafer bei 3.400 Umdrehungen aufgeschleudert. Nach 1 Minute Trocknen bei 100 °C wurde eine Schichtdicke von 0,98 »m erhalten.

Das Aufzeichnungsmaterial wurde bildmäßig unter einer Vorlage mit der Strahlung einer Xenon-Quecksilberdampflampe bei 240 bis 260 nm mit einer Energie von 89 mJ/cm² belichtet.

Entwickelt wurde das Aufzeichnungsmaterial mit einer 0,02 n wäßrigen Lösung von Tetramethylammoniumhydroxid, wobei die belichteten Bereiche innerhalb von 60 s rückstandsfrei abgelöst wurden.

Ein weiteres Mal wurde ein fehlerfreies Abbild der Maske mit steilen Resistflanken erhalten. Der Dunkelabtrag betrug weniger als 20 nm; auch Strukturen von weniger als 0,6 »m waren detailgetreu aufgelöst.

### Beispiel 9

Es wurde eine Beschichtungslösung hergestellt aus
7,5 Gt des in Beispiel 8 angegebenen Copolymeren und
2,0 Gt der Verbindung 11 in
42 Gt Cyclohexanon.

Die Lösung wurde durch ein Filter mit einem Porendurchmesser von 0,2 »m filtriert und auf einen mit einem Haftvermittler (Hexamethyldisilazan) behandelten Wafer bei 3.500 Umdrehungen aufgeschleudert. Nach 1 Minute Trocknen bei 100 °C wurde eine Schichtdicke von 1,04 »m erhalten.

Das Aufzeichnungsmaterial wurde bildmäßig unter einer Vorlage mit der Strahlung einer Xenon-Quecksilberdampflampe bei 240 bis 260 nm mit einer Energie von 67 mJ/cm² belichtet.

Entwickelt wurde das Aufzeichnungsmaterial mit einer 0,02 n wäßrigen Tetramethylammoniumhydroxidlösung, wobei die belichteten Bereiche innerhalb von 60 s rückstandsfrei abgelöst wurden und ein detailgetreues Abbild der Vorlage erhalten wurde. Die Kantensteilheit des Bildes war ausgezeichnet.

### Beispiel 10

Es wurde eine Beschichtungslösung hergestellt aus
7,5 Gt des in Beispiel 2 beschriebenen Copolymeren und
2,0 Gt der Verbindung 23 in
42 Gt Propylenglykol-monomethylether-acetat.

Die Lösung wurde durch ein Filter mit einem Porendurchmesser von 0,2 »m filtriert und auf einen mit einem Haftvermittler (Hexamethyldisilazan) behandelten Wafer bei 3.100 Umdrehungen aufgeschleudert. Nach 1 Minute Trocknen bei 100 °C wurde eine Schichtdicke von 1,07 »m erhalten.

Das Aufzeichnungsmaterial wurde bildmäßig unter einer Vorlage mit der Strahlung einer Xenon-Quecksilberdampflampe bei 240 bis 260 nm mit einer Energie von 75 mJ/cm² belichtet.

Entwickelt wurde das Aufzeichnungsmaterial mit einer 0,27 n wäßrigen Tetramethylammoniumhydroxidlösung, wobei die belichteten Bereiche innerhalb von 60 s rückstandsfrei abgelöst wurden und ein detailgetreues Abbild der Vorlage erhalten wurde. Linien und Spalten bis herab zu 0,5 »m wurden maskengetreu wiedergegeben. Es zeigte sich, daß die gefertigte Lösung des Materials auch nach 6-wöchiger Lagerung im Dunkeln noch reproduzierbare lithographische Ergebnisse lieferte, die mit den ersten Versuchen identisch waren.

### Beispiele 11 und 12 (Vergleichsbeispiele)

Die Resistformulierung des Beispiels 2 wurde derart abgeändert, daß der Mischung je 0,2 Gt der als Säurebildner bekannten Verbindungen Triphenylsulfonium-hexafluorphosphat (Beispiel 11) und 2-Nitrobenzyltosylat (Beispiel 12) zugesetzt wurden. Nach der Beschichtung und Trocknung zeigte sich, daß die Formulierung des Beispiels 11 gegenüber KrF-Excimer-Laserlicht einer Wellenlänge von 248 nm unter identischen Prozeßbedingungen um etwa 10% empfindlicher ist, als die erfindungsgemäße Mischung des Beispiels 2, während sich die Empfindlichkeit der Formulierung des Beispiels 12 nicht von der des Beispiels 2 unterscheidet.

Bei Verwendung des Onium-Salzes (Beispiel 11) wurden allerdings Strukturen mit sogenanntem "Lackfuß" erhalten, d.h. Resistreste haften in den belichteten Bereichen am Substrat an, während bei Verwendung des Tosylesters (Beispiel 12) Oberflächenvernetzungen sichtbar waren, die die freigelegten Substratflächen teilweise überdachten. In beiden Fällen waren somit keine akzeptablen Strukturierungen erhältlich.

## Patentansprüche

1. Verbindungen der allgemeinen Formel I 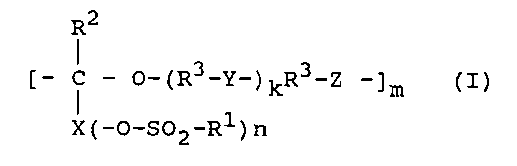 worin
R¹ einen Alkyl-, Halogenalkyl- oder Arylrest,
R² ein Wasserstoffatom, einen Alkyl-, Alkenyl- oder Arylrest oder die Gruppe (R¹-SO₂-O-)ₙX-,
R³ eine Cycloalkylendialkyl-, Cycloalkenylendialkyl-, Arylendialkyl- oder Heteroarylendialkylgruppe, eine Alkylen-, Alkenylen-, Alkinylen-, Cycloalkylen- oder Arylengruppe bedeutet,
X eine Alkyl-, Cycloalkyl- oder Arylgruppe, die mit 1 bis 3 Sulfonyloxygruppen R¹-SO₂-O- substituiert ist,
Y O, S, CO, CO-O, SO₂, SO₂-O, NR⁴, CO-NH, O-CO-NR⁵, NH-CO-NR⁵ oder NR⁵-CO-O,
Z O, CO-NR⁶, O-CO-NR⁶ oder NH-CO-NR⁶,
R⁴ einen Acylrest,
R⁵ ein Wasserstoffatom, einen Alkyl-, Cycloalkyl, Alkenyl-, Alkinyl- oder Arylrest,
R⁶ einen Alkyl-, Cycloalkyl, Alkenyl-, Alkinyl- oder Arylrest,
k eine ganze Zahl von 0 bis 4,
m eine ganze Zahl größer als 1 und
n eine ganze Zahl von 1 bis 3
bedeuten, wobei R³ und Y in sich wiederholenden Gruppierungen (R³-Y-) gleiche oder unterschiedliche Bedeutungen haben können.

2. Verbindungen nach Anspruch 1, dadurch gekennzeichnet, daß R¹ einen Alkyl-, hochfluorierten Alkyl- oder Perfluoralkylrest mit 1 bis 6 Kohlenstoffatomen oder einen Arylrest mit 6 bis 12 Kohlenstoffatomen bedeutet.

3. Verbindungen nach Anspruch 1, dadurch gekennzeichnet, daß X eine mit 1 bis 3 Sulfonyloxygruppen R¹-SO₂-O- substituierte Alkyl- oder Cycloalkylgruppe mit 2 bis 10 Kohlenstoffatomen oder eine derart substituierte Arylgruppe mit 6 bis 12 Kohlenstoffatomen bedeutet.

4. Positiv arbeitendes strahlungsempfindliches Gemisch, das im wesentlichen
a) ein in Wasser unlösliches, in wäßrig-alkalischen Lösungen lösliches oder zumindest quellbares Bindemittel und
b) eine unter Einwirkung von aktinischer Strahlung eine starke Säure bildende Verbindung mit mindestens einer durch Säure spaltbaren C-O-C-Bindung
enthält, dadurch gekennzeichnet, daß die Verbindung (b) eine Verbindung gemäß einem der Ansprüche 1 bis 3 ist.

5. Strahlungsempfindliches Gemisch nach Anspruch 4, dadurch gekennzeichnet, daß die Verbindung (b) gegenüber Licht einer Wellenlänge von 190 bis 350 nm empfindlich ist.

6. Strahlungsempfindliches Gemisch nach Anspruch 4, dadurch gekennzeichnet, daß es die Verbindung (b) in einer Konzentration von 2 bis 60 Gew.-% enthält.

7. Strahlungsempfindliches Gemisch nach Anspruch 4, dadurch gekennzeichnet, daß das Bindemittel (a) im Wellenlängenbereich der Empfindlichkeit der Verbindung (b) eine Extinktion von weniger als 0,5 »m⁻¹ aufweist.

8. Strahlungsempfindliches Gemisch nach Anspruch 7, dadurch gekennzeichnet, daß das Bindemittel ein Polymeres mit phenolischen Hydroxylgruppen ist.

9. Strahlungsempfindliches Gemisch nach Anspruch 4, dadurch gekennzeichnet, daß es das Bindemittel (a) in einer Konzentration von 40 bis 98 Gew.-% enthält.

10. Strahlungsempfindliches Aufzeichnungsmaterial aus einem Schichtträger und einer strahlungsempfindlichen Schicht, dadurch gekennzeichnet, daß die Schicht aus einem strahlungsempfindlichen Gemisch gemäß einem der Ansprüche 4 bis 9 besteht.

## Claims

1. A compound of the formula I 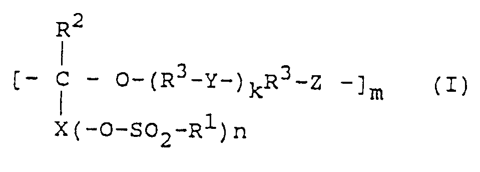 in which
R¹ is an alkyl, halogenoalkyl or aryl radical,
R² is a hydrogen atom, an alkyl, alkenyl or aryl radical or the group (R¹-SO₂-O-)ₙX-,
R³ is a cycloalkylenedialkyl, cycloalkenylenedialkyl, arylenedialkyl or heteroarylenedialkyl group, or an alkylene, alkenylene, alkynylene, cycloalkylene or arylene group,
X is an alkyl, cycloalkyl or aryl group substituted by 1 to 3 sulfonyloxy groups R¹-SO₂-O,
Y is O, S, CO, CO-O, SO₂, SO₂-O, NR⁴, CO-NH, O-CO-NR⁵, NH-CO-NR⁵ or NR⁵-CO-O,
Z is O, CO-NR⁶, O-CO-NR⁶ or NH-CO-NR⁶,
R⁴ is an acyl radical,
R⁵ is a hydrogen atom or an alkyl-, cycloalkyl, alkenyl, alkynyl or aryl radical,
R⁶ is an alkyl, cycloalkyl, alkenyl, alkynyl or aryl radical,
k is an integer from 0 to 4,
m is an integer greater than 1 and
n is an integer from 1 to 3,
where R³ and Y in recurring groupings (R³-Y-) can have identical or different definitions.

2. A compound as claimed in claim 1, wherein R¹ is an alkyl, highly fluorinated alkyl or perfluoro-alkyl radical having 1 to 6 carbon atoms or an aryl radical having 6 to 12 carbon atoms.

3. A compound as claimed in claim 1, wherein X is an alkyl or cycloalkyl group having 2 to 10 carbon atoms and substituted by 1 to 3 sulfonyloxy groups R¹-SO₂-O- or an aryl group having 6 to 12 carbon atoms and substituted in this way.

4. A positive-working radiation-sensitive mixture which contains essentially
a) a binder which is insoluble in water and soluble or at least swellable in aqueous alkaline solutions, and
b) a compound which generates a strong acid under the action of actinic radiation and which has at least one acid-cleavable C-O-C bond,
wherein the compound (b) is a compound as claimed in any of claims 1 to 3.

5. A radiation-sensitive mixture as claimed in claim 4, wherein the compound (b) is sensitive to light of a wavelength from 190 to 350 nm.

6. A radiation-sensitive mixture as claimed in claim 4, which contains the compound (b) in a concentration from 2 to 60% by weight.

7. A radiation-sensitive mixture as claimed in claim 4, wherein the binder (a) has an extinction of less than 0.5 »m⁻¹ in the wavelength region of the sensitivity of compound (b).

8. A radiation-sensitive mixture as claimed in claim 7, wherein the binder is a polymer having phenolic hydroxy groups.

9. A radiation-sensitive mixture as claimed in claim 4, which contains the binder (a) in a concentration from 40 to 98% by weight.

10. A radiation-sensitive recording material comprising a layer support and a radiation-sensitive layer, wherein the layer is composed of a radiation-sensitive mixture as claimed in any of claims 4 to 9.

## Revendications

1. Composés de formule générale I 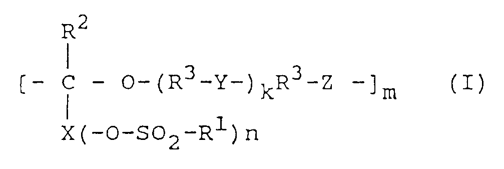 dans laquelle
R¹ représente un groupe alkyle, halogénoalkyle ou aryle,
R² représente un atome d'hydrogène, un groupe alkyle, alcényle ou aryle ou le groupe (R¹-SO₂-O-)ₙX-,
R³ représente un groupe cycloakylènedialkyle, cycloalcénylènedialkyle, arylènedialkyle ou hétéroarylènedialkyle, un groupe alkylène, alcénylène, alcynylène, cycloalkylène ou arylène,
X représente un groupe alkyle, cycloalkyle ou aryle, lequel est substitué par 1 à 3 groupes sulfonyloxy R¹-SO₂-O-,
Y représente O, S, CO, CO-O, SO₂, SO₂-O, NR⁴, CO-NH, O-CO-NR⁵, NH-CO-NR⁵ ou NR⁵-CO-O,
Z représente O, CO-NR⁶, O-CO-NR⁶ ou NH-CO-NR⁶,
R⁴ représente un groupe acyle,
R⁵ représente un atome d'hydrogène, un groupe alkyle, cycloalkyle, alcényle, alcynyle ou alkyle,
R⁶ représente un groupe alkyle, cycloalkyle, alcényle, alcynyle ou aryle,
k représente un nombre entier de 0 à 4,
m représente un nombre entier plus grand que 1 et
n représente un nombre entier de 1 à 3,
étant entendu que R³ et Y peuvent avoir des significations identiques ou différentes dans les motifs (R³-Y-).

2. Composés selon la revendication 1, caractérisés en ce que R¹ représente un groupe alkyle-, alkyle fortement fluoré ou alkyle perfluoré présentant de 1 à 6 atomes de carbone ou un groupe aryle présentant de 6 à 12 atomes de carbone.

3. Composés selon la revendication 1, caractérisés en ce que X représente un groupe alkyle ou cycloalkyle substitué par 1 à 3 groupes sulfonyloxy R¹-SO₂-O-, présentant de 2 à 10 atomes de carbone ou un groupe aryle substitué présentant de 6 à 12 atomes de carbone.

4. Composition photosensible travaillant en positif contenant essentiellement
a) un liant insoluble dans l'eau, soluble dans des solutions alcalines aqueuses ou au moins gonflables et
b) un composé formant un acide fort sous l'action d'un rayonnement actinique, présentant au moins une liaison C-O-C- clivable par un acide, caractérisée en ce que le composé (b) est un composé selon l'une des revendications 1 à 3.

5. Composition photosensible selon la revendication 4, caractérisée en ce que le composé (b) est sensible à une lumière de longueur d'ondes comprise entre 190 et 350 nm.

6. Composition photosensible selon la revendication 4, caractérisée en ce qu'elle contient le composé (b) dans une concentration comprise entre 2 et 60 % en poids.

7. Composition photosensible selon la revendication 4, caractérisée en ce que le liant (a) présente un coefficient d'extinction de moins de 0,5 »m⁻¹ dans la zone de longueur d'ondes à laquelle le composé (b) est photosensible.

8. Composition photosensible selon la revendication 7, caractérisée en ce que le liant est un polymère présentant des groupes hydroxyle phénoliques.

9. Composition photosensible selon la revendication 4, caractérisée en ce qu'elle contient le liant (a) dans une concentration comprise entre 40 et 98 % en poids.

10. Matériel de reproduction photosensible constitué d'une couche support et d'une couche photosensible, caractérisé en ce que la couche est constituée d'une composition photosensible selon l'une des revendications 4 à 9.
